# EUROPEAN PATENT APPLICATION

(11) **EP 2 053 127 A2**
(43) Date of publication of application: **29.04.2009**
(21) Application number: 08004452.2
(22) Date of filing: 06.12.2000
(51) Int. Cl.: C12N 15/67, C12N 9/38, C12N 15/56, A01K 67/027

(54) **Isolated polynucleotides having a reduced or an increased content of epigenetic control motifs and uses thereof**

(30) Priority: 06.12.1999 CA 2291367
(62) Divisional of application: 00991199.1
(71) Applicant: Institut Pasteur, 75724 Paris Cédex 15 (FR); Centre National de la Recherche Scientifique, 75794 Paris Cedex 16 (FR)
(72) Inventor: Nicolas, Jean-François, 78590 Noisy le Roi (FR); Henry, Isabelle, 92240 Malakoff (FR); Choulika, André, 75015 Paris (FR)
(74) Representative: Paris, Fabienne

(57) **Abstract**

The present invention is concerned with modified polynucleotides derived from a native gene and having a reduced or increased number of epigenetic control motifs, at the nucleotide level, as compared to the native gene. These polynucleotides are useful to study, increase and/or reduce genes expression, and to improve DNA vaccination methods. The present invention also relates to methods of using these modified polynucleotides in *in vitro* and *in vivo* expression systems.

## Description

### BACKGROUND OF THE INVENTION

### a) Field of the invention

The present invention is concerned with modified polynucleotides derived from a native gene and having a reduced or increased number of epigenetic control motifs, at the nucleotide level, as compared to the native gene. These polynucleotides are useful to study, increase and/or reduce genes expression, and to improve DNA vaccination methods. The present invention also relates to methods of using these modified polynucleotides in *in vitro* and *in vivo* expression systems.

### b) Brief description of the prior art

Epigenetic control nucleic acid sequences and regions are known to be involved in gene regulation expression.

In eukaryotes, numerous studies have shown that the methylation of 5'CpG3' dinucleotides (mCpG) has a repressive effect on gene expression in vertebrates and flowering plants (Hsieh, Mol. Cell. Biol., 14:5487-94. 1994; Kudo, Mol. Cell. Biol., 18:5492-99, 1998; Goto and Monk, Microbiol. Mol. Biol. Rev., 62:362-378, 1998; Jones et al. 1998, Collas 1998). The methylation of 5'CpG3' dinucleotides within genes creates potential targets for protein complexes that bind to methylated DNA sequences and to histone deacetylases (MBD-HDAC). This can lead to a transcriptional repression following modification(s) of the chromatin.

Up to now, the knowledge that methylation of CpG sequences within a gene dominantly silence transcription has been used to inhibit gene expression of genes that are over-expressed or for which expression is not desired. For example, U.S. patents Nos. 5,856,462 and 5,874,416 disclose oligonucleotides having a rich CpG dinucleotides content and anticipate the uses of these oligonucleotides for inhibiting specific gene expression.

Contrary to the prior art, the present invention aims to remove the inhibitory expression barrier which exists between organisms from different genus and species. This is achieved by modifying the content of the epigenetic regulation motif(s) which are known to be involved for blocking/stimulating the expression of genes in a particular host. With the present invention, it is possible to synthesize an artificial gene or a polynucleotide derived from the native gene of a first host and having at the nucleotide level a modified content of an epigenetic regulation motif specific to a second host and thereby modify accordingly the levels of expression of the artificial gene as compared to the unmodified native gene.

The present invention also fulfils other needs which will be apparent to those skilled in the art upon reading the following specification.

### SUMMARY OF THE INVENTION

The present invention is concerned with isolated polynucleotides derived from a native gene of a first host, the isolated polynucleotides having, at the nucleotide level, an increased or reduced content of at least one epigenetic regulation motif specific to a second host as compared to the native gene. The isolated polynucleotides thereby demonstrate a modified level of expression once introduced into a cell of the second host, as compared to the native gene's level of expression. Preferably, the sequence of the isolated polynucleotides according to the invention is such that levels of expression of the polynucleotides are increased into the second host, particularly in cases where, under standard conditions, the levels of expression of the native gene in the second host are nll or very low.

The present invention is also concerned with modified gene sequences having a lower or a higher content of at least one epigenetic regulation motif specific to a host expressing these genes.

In a preferred embodiment, the isolated polynucleotide derived from a prokaryotic gene, and its content of the at least one epigenetic regulation motif has been lowered for increasing its expression in an eukaryotic host.

In another preferred embodiment, the isolated polynucleotide derived from an eukaryotic gene, and its content of the at least one epigenetic regulation motif has been lowered for increasing its expression in an prokaryotic host.

The invention also encompasses expression vectors, cells, and living organisms genetically modified as to comprise and/or express any of the polynucleotides object of the invention. More particularly, the present invention provides two microorganisms having a modified LacZ gene with a lower CpG content. These microorganisms have been deposited at the Collection Nationale de Cultures de Microoganismes de l'Institut Pasteur (CNCM) under numbers I-1691 ("*pPytknlsLagZ*" deposited on April 16, 1996) and I-2354 ("*pBSEF LagoZ LTR*" deposited on November 25, 1999).

It is also an object of this invention to provide a method to express in a second host an isolated polynucleotide derived from a first host native gene sequence. This method is characterized in that it comprises the step of providing an isolated polynucleotide for which expression is desired by modifying the nucleic acid sequence of the native gene in order to modify its nucleotide content in at least one epigenetic regulation motif specific to the second host. The isolated polynucleotide is thereby capable of showing an increased level of expression when introduced into a cell of said second host as compared to the native gene level of expression in the same second host cell.

The invention covers also any modification in epigenetic nucleotidic control sequences which allows the expression of a purified polynucleotide in a second host which is a member of the same species of the first host. The use of an isolated polynucleotide according to the invention for compensating a genetic defect is also contemplated in the present invention.

Another object of this invention is to provide a method to measure expression levels of a gene having at least one epigenetic regulation motif. This method comprises the steps of:
a) providing a vector comprising a regulatory sequence and a reporter gene;
b) inserting into the vector a polynucleotide coding, or substantially complementary to, the gene for which expression is to be measured; this insertion being done between the regulatory sequence and the reporter gene of the vector;
c) inducing the expression of the inserted polynucleotide; and
d) assaying levels of expression of the gene.

This method is particularly useful for evaluating various promoters in various biological systems, for comparing methylation activity in different biological systems and/or for identifying unknown methyl DNA binding proteins.

The invention covers also the use of deprived or decreased amount of methylable epigenetic nucleotidic control sequences for the prevention of an immune response against exogenous DNA used in genetic or cellular therapy.

The invention and its numerous advantages will be better understood upon reading the following non-restrictive specification and the accompanying drawings which are for the purpose of illustration only.

### BRIEF DESCRIPTION OF THE DRAWINGS

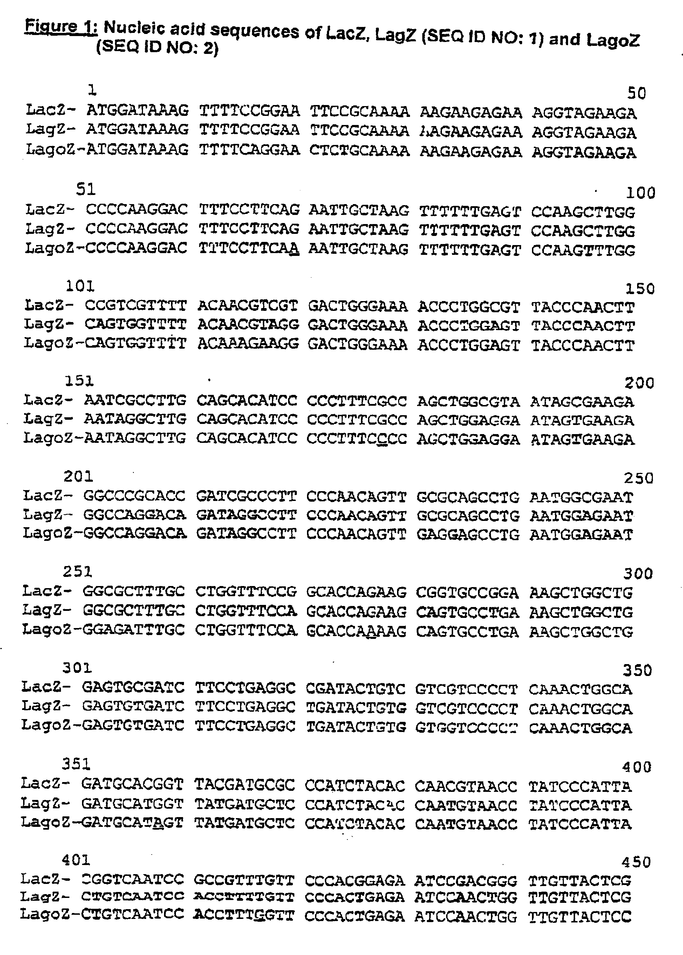

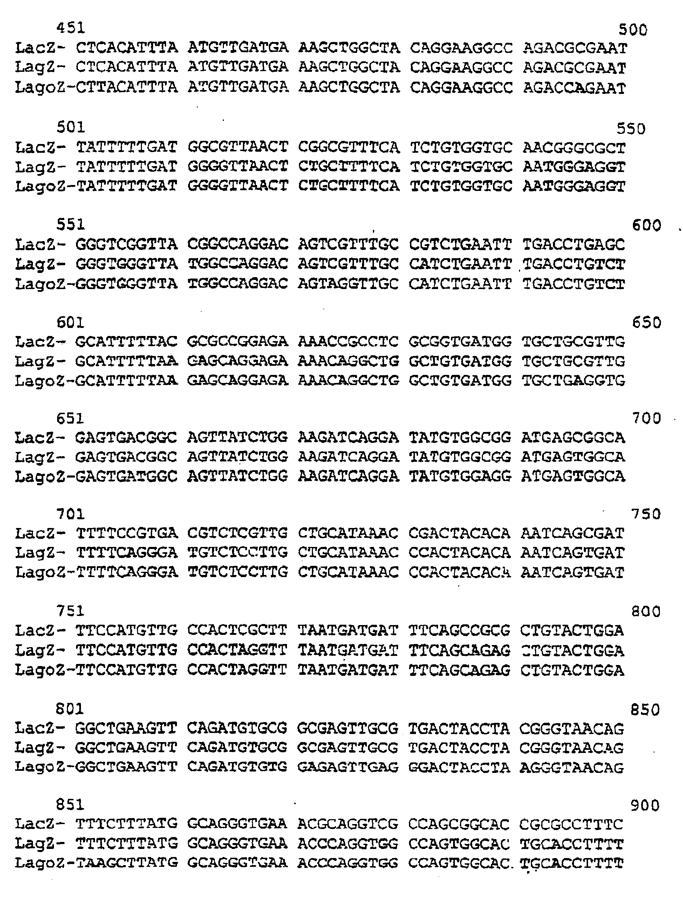

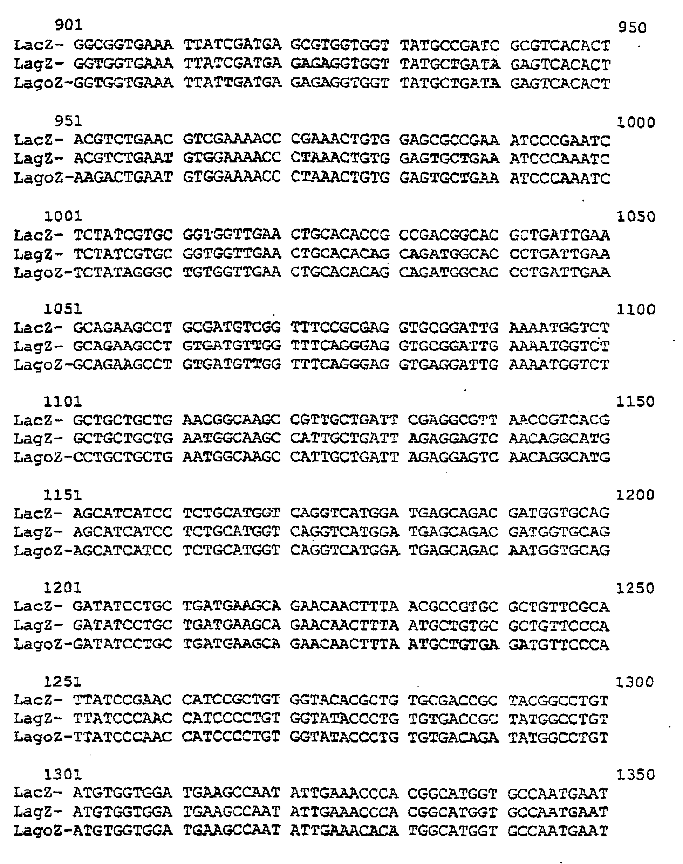

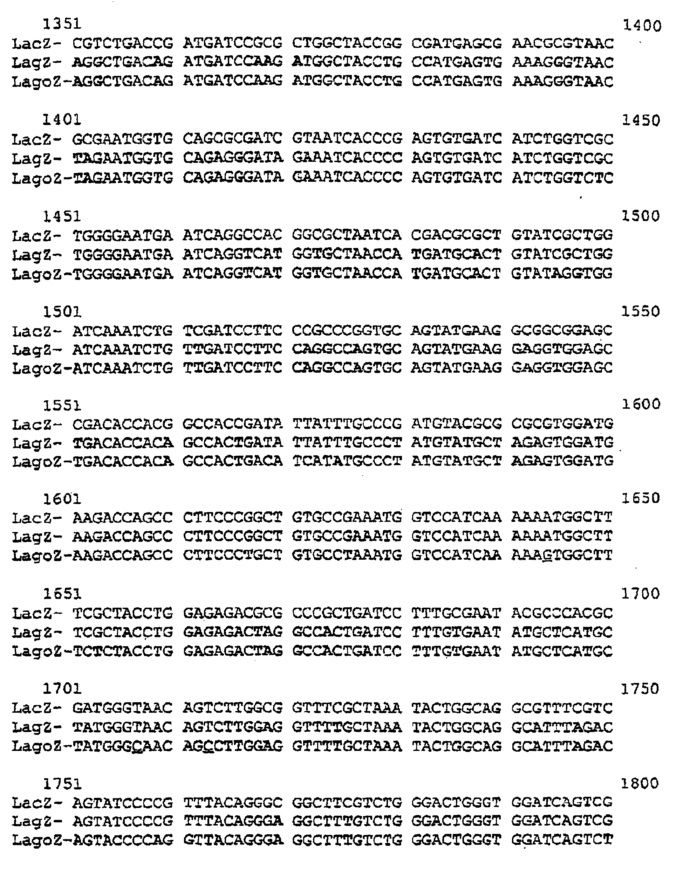

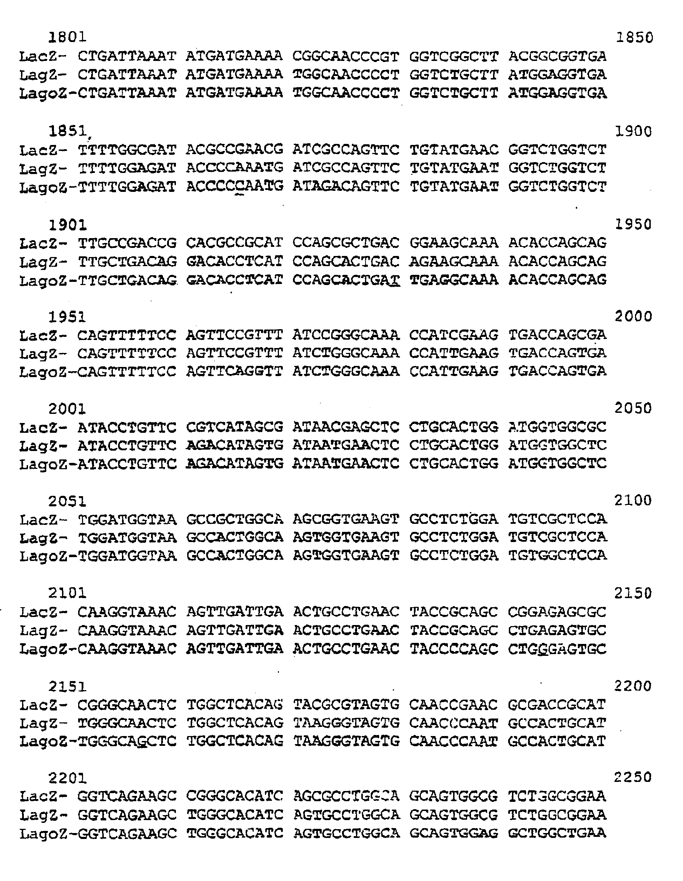

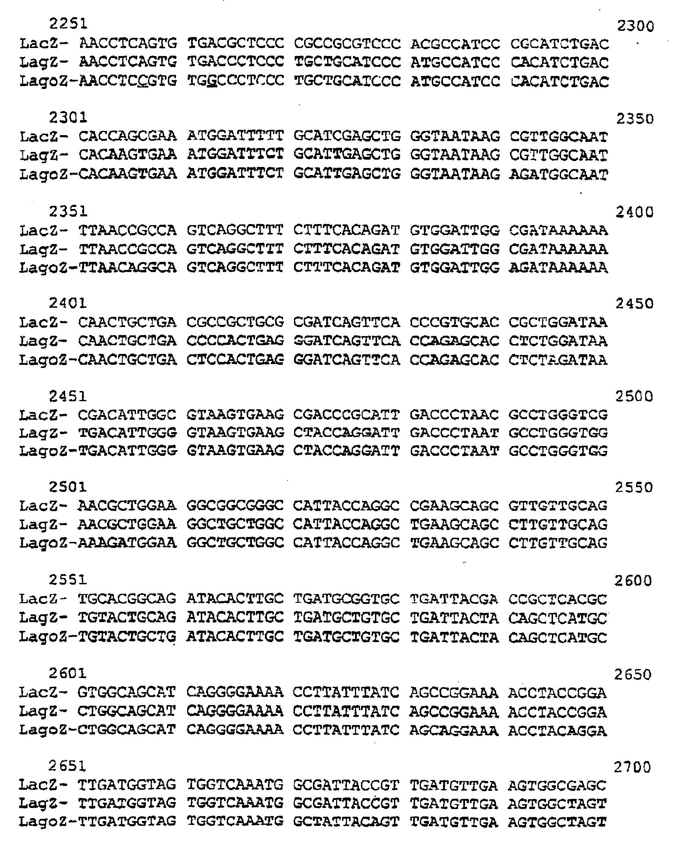

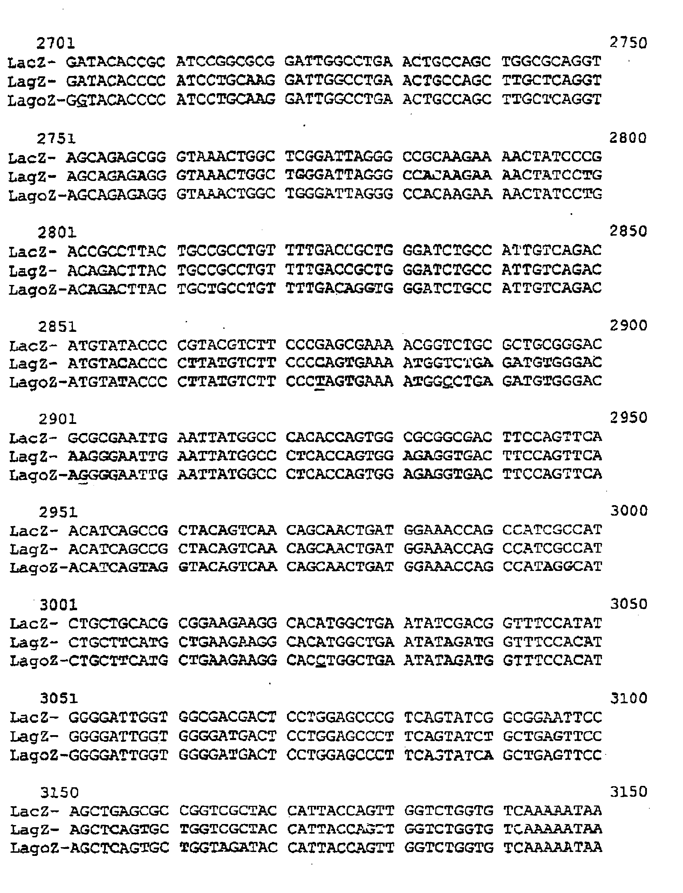

**Figure 1** shows the nucleotide sequences of LacZ, LagZ and LagoZ genes. Nucleotides in bold correspond to the conservative mutations introduced for changing CpG dinucleotides. Underlined nucleotides correspond to non-conservative fortuitous mutations which have appeared during mutagenesis cycles.

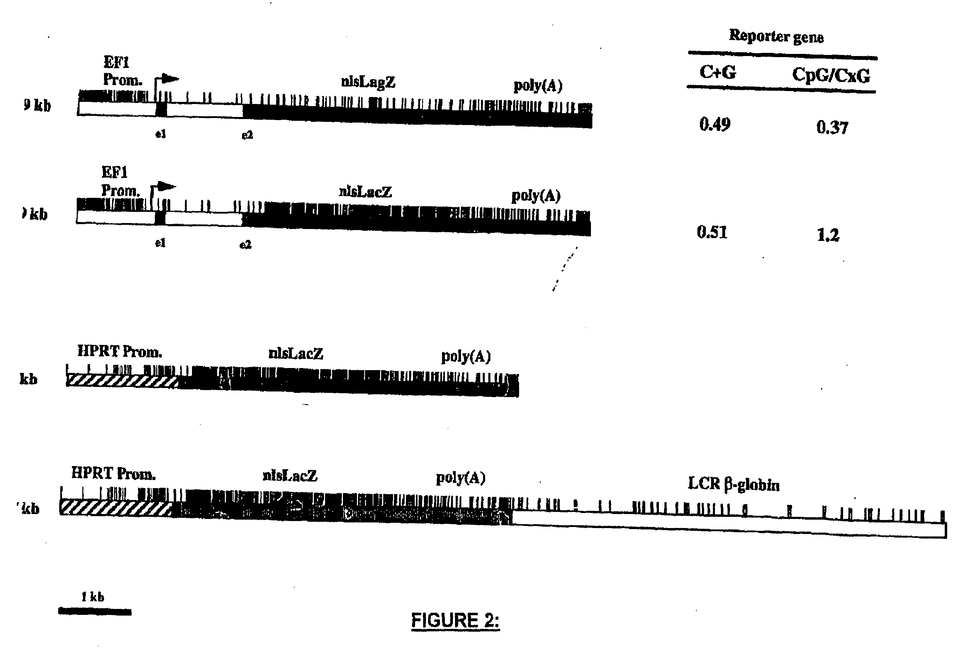

**Figure 2** shows the structure of DNA constructs that were made for generating transgenic mice expressing isolated polynucleotides according to the invention. All constructs contain the nuclear localization signal of SV40 (nls), a reporter gene, LagZ or LacZ gene and the MoMuLV polyadenylation signal. Each vertical dash above or below the reporter gene indicates a CpG dinucleotide. The size of each DNA Insert is indicated in kilobases (kb). *EF1*α *Prom.:* promotor of the human translocation elongation factor a subunit gene; *e1*: exon 1; *HPRT Prom*.: promotor of the human hypoxanthine phosphoribosyl transferase gene; *LCR* β*-giabin*: mini-locus control region of the β-globin locus: *Poly A*: the polyadenylation signal of Moloney murine leukemia virus. The table at the left side contains parameters used to identify a CpG rich region according to Larsen et al. (1992) for each reporter gene. *(C*+*G)* is the (number of C plus the number of G)/number of nucleotides in the sequence, and *CpG*/*CxG* is the (number of CpG x number of nucleotides in the sequence)/(number of C x number of G).

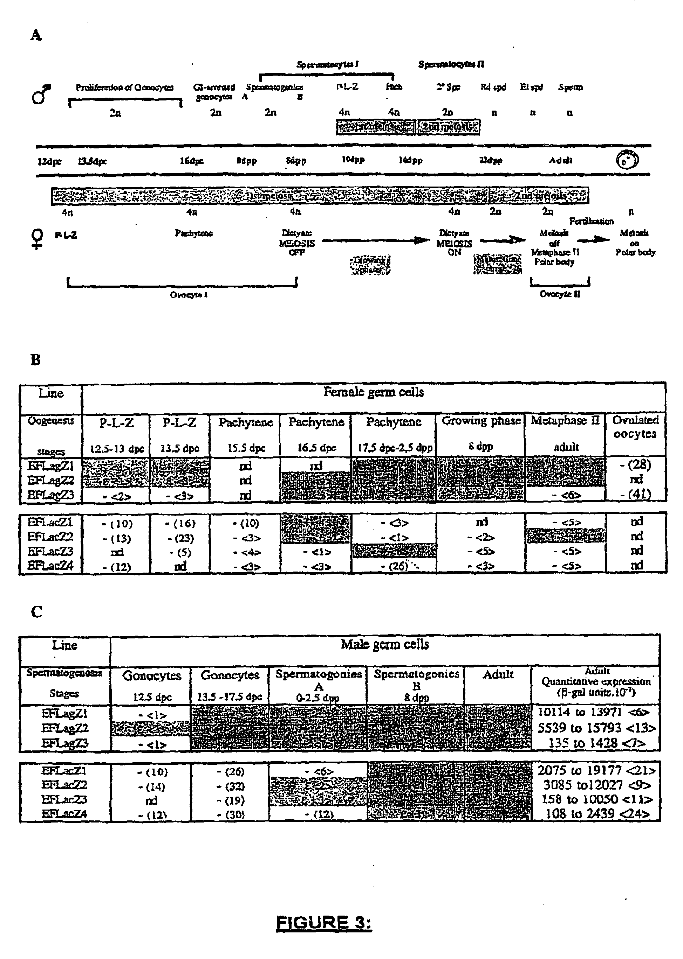

**Figure 3** shows results of the expression of EFLagZ and EFLacZ transgenes expression during gametogenesis.
   **A)** Chronology of meïotic events during oogenesis and spermatogenesis
      The figure summarizes the timing of important events in oogenesis and spermatogenesis, beginning from the stage of colonization in the genital ridges by primordial germ cells to the stage of mature gametes and the first stage cleavage after fertilization. *dpc* refers to the number of after mating (for embryos), *dpp* indicates the number of days after birth. *P*, *L*, *Z*: preleptotene, leptotene and zygotene stages of prophase 1, respectively. *Pach*: pachytene stage of prophase 1; 2° *Spc*: secondary spermatocyte; Rd *spd*: round spermatid; *EI spd*: elongated spermatid and *n*: haploïd genome.
   **B)** Expression of maternally and **C)** paternally transmitted EFLagZ and EFLacZ transgenes during gametogenesis and in the adult gonad
      Embryos or animals were obtained by crossing heterozygous transgenic females or males to (B6D2)F1 males or females according to the parental origin of the transgene. Numbers between arrows indicate the number of analyzed embryos or animals. - : β-gal negative; +: β-gal positive; *e*: only a few germ cells were β-gal positive; *: β-gal positive germ cells were clustered, *1*: one transgenic female was β-gal positive in gonads; *2*: two transgenic females were β-gal positive in gonads; 3: four transgenic females were β-gal positive in gonads; *4*: two transgenic males were β-gal positive in gonads; *nd*: not determined. The last column in C) refers to a quantitative analysis of parental transgene expression in adult testis. The β-gal activity was quantified using a fluorogenic substrate of β-galactosidase (MUG). β-gal activity of control testis was 41.5 x 10⁻⁷ β-gal units (mean value of 12 control testes were analyzed).

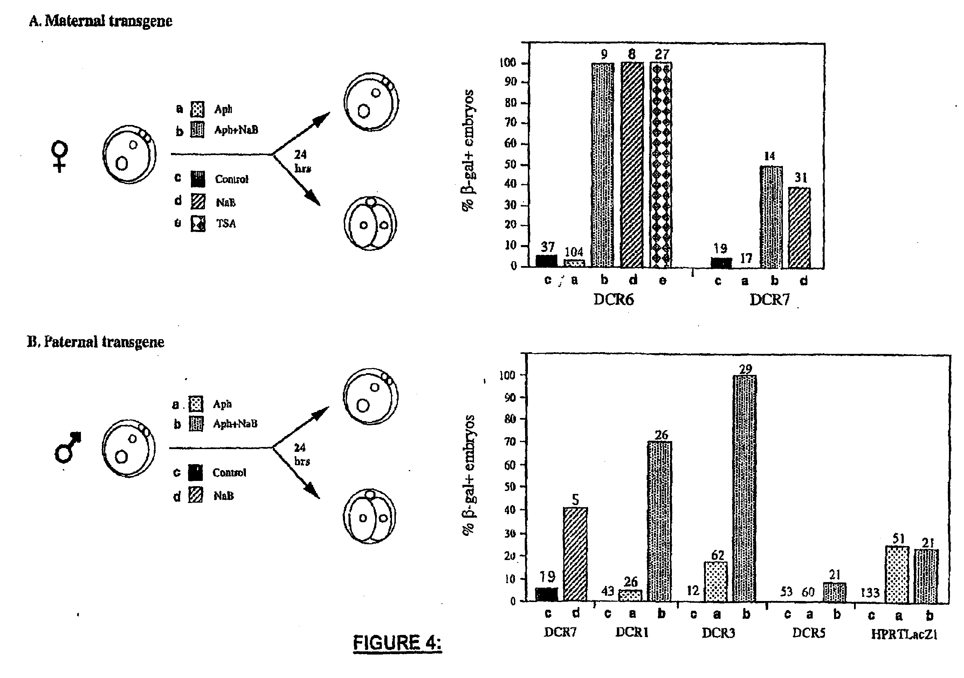

**Figure 4** shows results indicating that inhibitors of histone deacetylases relieve the repressed state of maternally and paternally transmitted LacZ transgenes in 2-cell embryos. One-cell embryos from different lines, carrying a transgene of maternal **(A)** or paternal **(B)** origin were recovered at 24 hphCG and allowed to develop in the absence (control) or presence of sodium butyrate (NaB; 2.5 mM) or trichostatin A (TSA; 66 nM) for 24 h. Aphidicolin, an inhibitor of DNA polymerases was used alone (Aph; 2 µg/ml) or in combination with sodium butyrate (Aph + NaB).

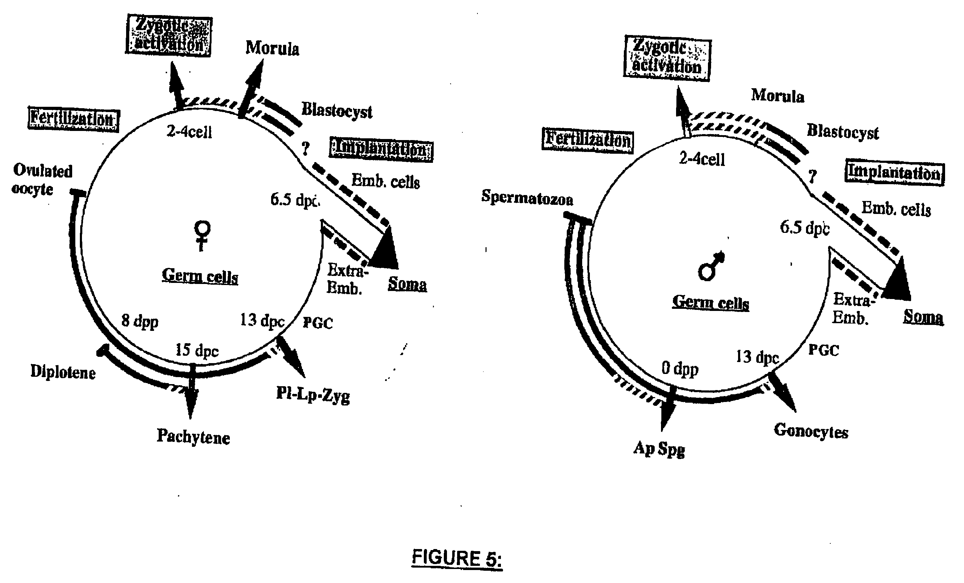

**Figure 5** summarizes in diagrams the expression of EFLagZ, EFLacZ, HPRTLacZ and HPRTLacZDCR transgenes during gametogenesis and early development of the embryo. EFLagZ and LacZ transgenes expression during gamete and embryo development is indicated as red and green draws respectively. In the left part is indicated transgenes expression through a paternal genome and in the right part, transgenes expression through the maternal genome. Gametogenesis is shown at the bottom of the cycle, the cleavage period of the embryo is shown at tne top of the cycle and the post-implantation embryo is shown to the right. Periods of development at which the transcriptional permissiveness of transgenes changes is indicated by the arrows. Stages of gametogenesis and the embryo at preimplantation corresponding to the relief of transgene inhibition (red, green and black arrows) of the establishment of inhibition (red, green and black vertical bars) are indicated outside the cycle. Red and green dashed lines indicate that the relief of transgene inhibition is progressive. Black vertical bars and arrows indicate that the two EFLagZ and LacZ transgenes were inhibited and become expressed in the same cell type or preimplantation period. *dpc*: day post-coïtum; dpp: day post-partum; *PGC*: primordial germ cells; *Ap Spg*: type A spermatogonies; *PI-Lp-Zyg*: preleptotene, leptotene and zygotene stages of prophase I.

### DETAILED DESCRIPTION OF THE INVENTION

The present invention first aims at removing the inhibitory expression barrier of genes, and more particularly between genes of hosts from different genus or species.

In order to provide an even clearer and more consistent understanding of the specification and the claims, including the scope given herein to such terms, the following definitions are provided:

### A) Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described. All U.S. patents and scientific literature cited in this application evidence the level of knowledge in this field and are hereby incorporated by reference. For purposes of clarification, the following terms are defined below.

**Derived:** (see also "modified"). A polynucleotide is said to "derive" from a native gene or a fragment thereof when such polynucleotide comprises at least one portion, substantially similar in its sequence, to the native gene or to a fragment thereof. Preferably, the polynucleotide is also similar in its function to the native gene from which it derives.

**Expression:** The terms "expression" or "expressing", as is generally understood and used herein, refer to the process by which a structural gene produces a polypeptide. It involves transcription of the gene into mRNA, and the translation of such mRNA into polypeptide(s).

**Epigenetic:** Means any change of the DNA structure, the chromatin or of the RNA which does not involve modifications of the nucleotides comprising the DNA or RNA. These changes can lead to the tri-dimensional modifications in DNA or chromatin structure. Examples of changes include chemical modifications of the purines or the purimydines constituting the DNA.

**Epigenetic regulation:** Means all chemical modifications introduced by a host cell against a natural or artificial DNA sequence. It also means chromatin structure modifications that a host cell inflicts to a natural or artificial DNA sequence. It also includes compartmentalization of a natural or artificial DNA sequence within a nuclear compartment of a celi comprising particular transcriptional and chromatinic properties. In eukaryotes, a well known epigenetic regulation motif is the 5'CpG' dinucleotides which can be methylated or unmethylated and thereby regulates transcription of a gene. In prokaryotes, a known epigenetic regulation motif includes the sequence 5'GATC3'.

**Expression vector:** Refers to a vector or vehicle similar to a cloning vector but which is capable of expressing a gene (or a fragment thereof) which has been cloned therein. Typically, expression of the gene occurs when the vector has been introduced into the host. The cloned gene is usually placed under the control of certain control sequences or regulatory elements such as promoter sequences. Expression control sequences vary depending on whether the vector is designed to express the operable linked gene in a prokaryotic or eukaryotic host and may additionally contain transcriptional elements such as enhancer elements, termination sequences, tissue-specificity elements and/or translational and termination sites.

**Fragment:** Refers to any part of a gene or a polynucleotide which is sufficient to encode a whole polypeptide, one of its portion or one of its epitope.

**Gene:** A nucleic acid molecule which is transcribed (in the case of DNA) and translated (in the case of mRNA) into a polypeptide *in vitro* or *in vivo* when placed under the control of appropriate regulatory sequences. The boundaries of the gene are normally determined by a start codon at the 5' (amino) terminus and a translation stop codon at the 3' (carboxy) terminus. A gene can include, but is not limited to, cDNA from prokaryotic or eukaryotic mRNA, genomic DNA sequences from prokaryotic or eukaryotic DNA, and even synthetic DNA sequences. A transcription termination sequence will usually be located 3' to the gene sequence. However, the invention not restricted to whole genes only since, depending of particular uses, fragments of gene and/or chimeric genes could also be used.

**Host:** A cell, tissue, organ or organism capable of providing cellular components for allowing the expression of an exogenous nucleic acid embedded into a vector or a viral genome, and for allowing the production of viral particles encoded by such vector or vira! genome. This term is intended to also include hosts which have been modified in order to accomplish these functions. Bacteria, fungi, animal (cells, tissues or organisms) and plant (cells, tissues, or organisms) are examples of a host. "Non-human hosts" comprise vertebrates such as rodents, non-human primates, sheep, dog, cow, amphibians, reptiles, etc.

**Isolated:** Means altered "by the hand of man" from its natural state, i.e., if it occurs in nature, it has been changed, purified or removed from its original environment, or both. For example, a polynucleotide naturally present in a living organism is not "isolated". The same polynucleotide separated from the coexisting materials of its natural state, obtained by cloning, amplification and/or chemical synthesis is "isolated" as the term is employed herein. Moreover, a polynucleotide that is introduced into an organism by transformation, genetic manipulation or by any other recombinant method is "isolated" even if it is still present in said organism.

**Modified:** As used herein, the terms "modified", "modifying" or "modification" as applied to the terms polynucleotides or genes, refer to polynucleotides that differ, in their nucleotide sequence, from another reference polynucleotide or gene. Changes in the nucleotide sequence of the modified polynucleotide may or may not alter the amino acid sequence of a polypeptide encoded by the reference polynucleotide/gene. Nucleotide changes may result in amino acid substitutions, additions, deletions, fusion proteins and truncations in the polypeptide encoded by the reference sequence. According to preferred embodiments of the invention, the modifications are conservative such that these changes do not alter the amino acid sequence of the encoded polypeptide. Modified polynucleotides may be made by mutagenesis techniques, by direct synthesis, and by other recombinant methods known to the skilled artisans. The polynucleotides of the invention can also contain chemical modifications or additional chemical moieties not present in the native gene. These modifications may improve the polynucleotides solubility, absorption, biological half life, and the like. The moieties may alternatively decrease the toxicity of the polynucleotides, eliminate or attenuate any undesirable side-effects and the like. A person skilled in the art knows how to obtain polynucleotides derived from a native gene.

**Native:** As used herein as applied to an object, refers to the fact that an object can be found in nature. For example, a gene that is present in and organism that can be isolated from its natural non-isolated state is said to be a "native gene".

**Polynucleotide:** Any DNA, RNA sequence or molecule having one nucleotide or more, including nucleotide sequences encoding a complete gene. The term is intended to encompass all nucleic acids whether occurring naturally or non-naturally in a particular cell, tissue or organism. This includes DNA and fragments thereof, RNA and fragments thereof, cDNAs and fragments thereof, expressed sequence tags, artificial sequences including randomized artificial sequences.

**Vector:** A self-replicating RNA or DNA molecule which can be used to transfer an RNA or DNA segment from one organism to another. Vectors are particularly useful for manipulating genetic constructs and different vectors may have properties particularly appropriate to express protein(s) in a recipient during cloning procedures and may comprise different selectable markers. Bacterial plasmids are commonly used vectors.

### B) General overview of the invention

The invention is based on the use of isolated polynucleotides derived from a native gene of a first host and having, at the nucleotide level, an increased or reduced content of at least one epigenetic regulation motif specific to second host, as compared to the native gene. These polynucleotides therefore demonstrate a modified level of expression once introduced into a cell of the second host, as compared to the native gene's level of expression.

For instance by deleting, in the gene sequence of a gene from the host "Y", at least some of the epigenetic regulation motif(s) which are known to act in the host "X" as specific blocking factors for the expression of natural gene(s), one can increase the expression of the "Y" gene in host "X". Similarly, if the epigenetic regulation motif(s) in the host "Y" are known to increase the expression of its genes, one can modify the gene sequence of a gene from the host "X" and increase the expression of this gene in the nost "Y".

In one aspect, the present invention relates to isolated polynucleotides derived from a native gene of a first host, with a modified content, at the nucleotide level, of at least one epigenetic regulation motif specific to a second host, as compared to the native gene. Under suitable expressing conditions, these polynucleotides demonstrate a modified level of expression once introduced into a cell of the second host, as compared to the native gene's level of expression. Preferably, the content of the epigenetic regulation motif(s) is modified so as to increase the level of expression of the polynucleotides.

As a specific example of a suitable oligonucleotide according to the invention is a polynucleotide deriving from prokaryotic gene, which number of 5'CpG3' dinucleotides has been lowered of up to 99.3% for increasing its expression in an eukaryotic host. The following examples describe two modified LacZ genes from a bacterial source, namely "*LagZ*" and '*LagoZ* having respectively 52 and 2 CpG dinucleotides as compared to the 291 CpG found in the native LacZ gene. These genes were found to have an increased expression in mouse embryos as compared to the unmodified LacZ gene.

Another specific example of a suitable oligonucleotide according to the invention is a polynucleotide deriving from eukaryotic gene, which content in 5'CpG3' dinucleotides has been increased for allowing/increasing its expression in an prokaryotic host. Such oligonucleotides could code for highly valuable proteins for which high levels of expressions in bacteria is desired such as genes coding angiogenic proteins such as VEGF, endostatine or angiostatine; and genes coding growth factors such as GMCSF.

A person skilled in the art will however understand that the invention is not limited to modifications is CpG motifs. It encompasses also other known epigenetic regulation motifs are such as 5'GATC3' in prokaryotes and any other sequence involved in the conformation changes of the chromatin.

In further aspects, the present invention relates to expression vectors, cells, and living organisms genetically modified to comprise and/or express any of the isolated polynucleotides according to the invention. "Genetically modified" cells and living organisms would preferably integrate and express a foreign DNA inserted therein. Well known methods for reliably inserting a foreign DNA into cells and/or living organisms include: bacterial transformation, transgenesis, stem cells transformation, viral transfection, and artificial chromosome insertion. Once inserted, the foreign DNA may be found integrated to the genome of the host or be found under a non-integrated form (episomal, plasmidic or viral). It may also be inserted to an artificial chromosome or to an independent genome such as into the genome of a bacterial parasitizing an eukariotic cell. In a preferred embodiment, the invention relates to microorganisms with a modified LacZ gene having a lower CpG content, and more particularly, to the microorganisms deposited at the CNCM under numbers I-1691 and I-2354.

In another aspect, the present invention relates to a method to express in a second host an isolated polynucleotide derived from a first nost native gene sequence. The method comprises the step of providing an isolated polynucleotide for which expression is desired by modifying the nucleic acid sequence of the native gene in order to modify its nucleotide content in at least one epigenetic regulation motif specific to the second host. The isolated polynucleotide thereby shows an increased level of expression when introduced into a cell of the second host as compared to the native gene level of expression in the second host. The method generally also comprises the step of introducing the isolated polynucleotide into the second host using a method preferably selected from the group comprising transgenesis, viral transfection, bacterial transformation, artificial chromosome insertion or homologeous recombination as disclosed for example by Cappuchi et al. (Trends genetics, 1989, 5:70-76) or by Brulet *et a*/. in European Patent No. 419 621.

Preferably, the nucleic acid sequence modifications are conservative modifications so that the amino acid sequence of the protein/polypeptide expressed by the native gene remains unchanged. Even more preferably, the epigenetic regulation motif comprises 5'CpG3' dinucleotides and the host is an eukaryote.

In still another aspect, the present invention relates to a method to measure expression levels of a gene having at ieast one epigenetic regulation motif, the method comprising the steps of:
a) providing a vector comprising a regulatory sequence and a reporter gene;
b) inserting into the vector a polynucleotide coding, or substantially complementary to, the gene for which expression is to be measured; such insertion being done between the regulatory sequence and the reporter gene of the vector;
c) inducing the expression of the inserted polynucleotide; and
d) assaying levels of expression of said gene.

Typically, steps c) and d) are done once the vector has been introduced into a suitable host. This method is particularly useful for evaluating promoter in biological systems, for comparing methylation activity in biological systems and/or for identifying unknown methyl DNA binding proteins.

In theory, the principles of the present invention could also be used, and are within the scope of the present invention, to reduce and even silence the expression of specific genes. By adding epigenetic regulation motif(s) in a polynucleotide derived from a native gene and inserting such polynucleotide in a cell, one can decrease or shut off expression of cis-genes proximal or distal to where the polynucleotide has integrated. This can be very useful for therapeutic applications, in cancer for example.

Yet, another aspect of the present invention is to provide a methods to express or to silence a gene sequence or a fragment thereof in a host cell *in vitro* or *in vivo*, the method comprising the steps of:
a) modifying an isolated nucleotide sequence of a gene for which *in vitro* or *in vivo* expression is desired by lowering the nucleotide content of this isolated gene in at least one epigenetic regulation motif, the epigenetic regulation motif being specific to the host cel' in which *in vitro* or *in vivo* expression is desired;
b) inserting into the host cell the isolated and modified gene sequence of step a);
c) inducing the expression of the isolated and modified gene sequence of step b).

A similar method can be used to reduce or silence the expression of a gene sequence in a host cell *in vitro* or *in vivo,* the method comprising the steps of:
a) modifying an isolated nucleotide sequence of a gene for which *in vitro* or *in vivo* reduction of expression or silencing is desired by lowering the nucleotide content of this isolated gene in at least one epigenetic regulation motif, the epigenetic regulation motif being specific to the host cell in which *in vitro* or *in vivo* reduction of expression or silencing is desired;
b) inserting into the host cell the isolated and modified gene sequence of step a);
c) reducing or silencing the expression of the isolated and modified gene sequence of step b) or of a cis-gene proximal or distal to the modified gene sequence inserted in b).

In preferred embodiments of these methods, the modifications onto the isolated gene sequence are 5'CpG3' dinucleotides conservative modifications which are introduced using directed mutagenesis methods.

In still another aspect, the present invention provides a method to compare the methylation activity in a biological system and/or identify unknown methyl DNA binding proteins. Such method may be used to measure the expression levels of a gene having at least one epigenetic regulation motif, by using a vector having a regulatory sequence and a reporter gene. More particularly, this method comprises the steps of:
a) inserting into the vector a polynucleotide sequence, a gene sequence, or a sequence substantially complementary to a gene having at least one epigenetic regulation motif and for which expression is to be measured, the insertion being done between the regulatory sequence and the reporter gene of the vector;
b) inducing the expression of the polynucleotide sequence, gene sequence, or complementary sequence; and
c) assaying the levels of expression of the gene.

Another aspect of the present invention is the use of an isolated polynucleotide, derived from a native gene and modified by changing the percentage of epigenetic regulation nucleotidic sequences motif, in the induction of a protective immune response *in vivo* or *in vitro.* The administration of such isolated oligonucleotide may help and increase the use of the DNA vaccine methods *in vivo.* A better T-cell response could also be envisaged by an *in vitro* stimulation of lymphocytes of a patient against a non-natural polynucleotide of interest according to the invention, as compared to the T-cell response against a natural native polynucleotide.

More particularly, the method of the invention for inducing in a second host, a protective immune response *in vivo* or *in vitro,* against a gene product of a first host, could comprises the following steps:
a) preparing at least one polynucleotide derived from the gene of a first host according;
b) administering at least one polynucleotide of step a) or a fragment thereof to the second host; and optionally,
c) measuring the immune response obtained against said gene product.

The invention is also concerned with the use of deprived or decreased amount of methylable epigenetic control sequences for the prevention of autoimmune against endogenous methyl CpG motifs, DNA used in genetic or cellular therapy or any host similar sequences. Indeed, isolated polynucleotides of the invention with no or a reduced number of ep.genetic nucleotidic control sequences, fragments thereof or vectors containing them, could be used to minimize a T-cell response against the T-cells or tissues treated with them. The invention thus proposed a new concept of DNA vaccination based on lowering/deleting epigenetic nucleotidic control sequences of a whole polynucleotide encoding an antigen, or only on a portion thereof, the modified polynucleotide still encoding an immunoactive antigen.

The following examples are intended to further illustrate certain preferred embodiments of the invention and are not intended to limit the scope of the invention.

### EXAMPLE 1:

### ENGINEERING AND EXPRESSION OF TWO MODIFIED LacZ GENES

### 1.0 Introduction

Methylation of the 5-position of the cytosine residues in the DNA is associated with transcriptional repression In vertebrates and flowering plants. Methylcytosine-binding proteins (MDB) possess a transcriptional repressor domain that binds co-repressors that include histone deacetylases (HDAC). These multiprotein complexes can be incorporated into nucleosomes. Acetylation of lysine residues on histones H2A, H2B, H3 and H4 has a permissive role to control the access of transcriptional activators to nucleosomes. Histone acetyl-transferases are frequently coactivators of transcription. Many experiments have demonstrated that methylation of CpG sequences within a gene dominantly silences transcription through the assembly of a repressive nucleosomal array. This repression can be relieved by inhibitors of histone deacetylases such as trichostatin A (TSA) or sodium butyrate (NaB) or by demethylation drugs such as 5-azacytidine. Therefore, it establishes a direct causal relationship between DNA methylation-dependent transcriptional silencing and the modification of chromatin through histone acetylation/deacetylation. DNA methylation is probably involved in silencing of transposable elements, retrotransposons and proviral DNA as a host defense function which inactivates parasitic sequences. DNA methylation is also directly involved in parental genomic imprinting and promoter inactivation at the origin of certain cancers. Finally, methylation is required for mammalian development because embryos that cannot maintain normal methylation levels die after gastrulation. The mammalian genome contains both CpG rich and CpG poor regions. Those rich in CpG, called CpG islands, are often associated with the promotor of genes, and are generally unmethylated. Those poor in CpG are generally methylated. So far, there is no specific role or property associated with either of these two types of regions.

If the m⁵CpG sequences complexed with MBD-HDAC are potent transcriptional repressors, then the natural or artificial insertion of DNA sequences into the genome (or other genetic modifications such as translocations or deletions) leading to a new distribution or to creation of CpG rich regions could lead to conditions of epigenetic silencing. For instance, the introduction of CpG rich bacterial genes or of artificial cDNAs into the genome could induce their silencing. However, there is no direct demonstration *in vivo* of either the repression of an endogenous gene by methylation or of any of these speculations.

A direct and simple way to demonstrate this and to test these speculations would be to compare the expression of molecules differencing only in their CpG content. We describe here two molecules modified from the CpG rich (9.24%) bacterial LacZ gene. These two molecules, called LagZ and LagoZ, have a CpG content of 1.65% (close to the value of vertebrate genomes outside the CpG island) and 0.06 %, respectively. They encode the same β-galactosidase as LacZ, therefore the expression of the gene can be followed in individual cells in the intact organism. Thus, these molecules could form the basis of a powerful system to answer fundamental questions concerning epigenetic controls apposed on genes during development and gametogenesis.

### 2.0 Material and Methods

### 2.1 Directed mutagenesis

Replacement of the CpG dinucleotides from the LacZ sequence consisted in the PCR amplification of a plasmid comprising the gene nlsLacZ (Bonnerot et al., 1987) and of the gene nlsLagZ using a pair of primers comprising the desired mutations. PCR reactions were done using 1ng of plasmidic DNA in a buffer: 50 mM Tris-HCl (pH 8.8), 150 µg/ml BSA, 16 mM (NH₄)₂SO₄ , 4.5 mM MgCl₂, 250µM of each of dNTP, 1.25 U of DNA Taq Polymerase (CETUS^{™}), 0.078 U of Pfu DNA Polymerase^{(exo+)} (STRATAGENE^{™}), 20 pmoles per pair of nucleotidic primers. Amplification was done for 30 cycles (1 min 94°C, 1 min 65°C, 6 min 72°C). The band corresponding to the amplification product was then isolated from the 1% agarose gel, purified and recircularized. To do so, the PCR product was treated for 15 min at 12°C with 100µM of each dNTP, 5 U of T4 DNA Polymerase (USB), in a buffer comprising 50 mM NaCl, 10 mM Tris-HCl, 10 mM MgCl₂, 1mM dithiotreitol (pH 7.2), 50 µg/ml BSA. Next, the DNA was phosphorilated 30 min at 37°C in 30 mM ATP, 30 U of polynucleotide kinase (Biolabs), in a buffer comprising 50 mM NaCl, 10 mM Tris-HCl (pH 7 8), 10 mM MgCl₂, 10 mM dithiotreitol , 25 µg/ml BSA. The ligation was done overnight at 16°C in 20 mM ATP, 5 U Weiss of T4 DNA ligase, 50 mM mM Tris-HCl (pH 7.8), 10 mM MgCl2, 10 mM dithiotreitol , 25 µg/ml BSA. The ligation's product is then used to transfect bacteria by electroporation. Bacteria expressing a functional β-galactosidase were isolated. The plasmidic DNA was digested with restriction enzymes which allow the selection of mutated clones.

### 2.2 Transitional expression of LacZ, LagZ and LagoZ genes

The various constructions were microinjected in 1-cell stage mouse embryos according to a known protocol (Bonnerot and Nicolas, 1993). Embryos were cultured 24 hours and β-galactosidase activity was measured according to the "MUG" technique described by Forlani and Nicolas (Trends Genet. 1996).

### 3.0 Results

Figure 1 is a comparison of the LacZ gene sequence with the LagZ (SEQ ID NO: 1) and LagoZ (SEQ ID NO: 2) gene sequences which were obtained by directed mutagenesis. Analysis of these sequences shows that no (A+T) nor (C+G) regions were created during mutagenesis. However, many mutations appeared during the many PCR cycles. Eleven of these mutations have resulted in amino acid substitution. These substitutions are: E(24) ->K, E(93) ->K, K(120) - >S, F(139) ->L, R(432) ->G, T(644) ->I, E(715) ->G, S(753) ->R T(755) ->A, D(901) ->G, R(968) ->G. As we can see, no punctual mutation occurred in the domain extending from nucleotide 1341 to nucleotide 2076. This area thus appears to be non flexible since any modification therein suppresses the β-galactosidase enzymatic activity.

Two microorganisms comprising the LagZ and the LagoZ were prepared by transforming *E*. *coli* XL1 blue cells with the plasmids according to the invention using standard protocols and conditions. The transformed *E*. *coli* XL1 cells were deposited at the Collection Nationale de Cultures de Microoganisme de l'Institut Pasteur (CNCM) under numbers I-1691 (*pPytknlsLagZ* deposited on April 16, 1996) and I-2354 (*pBSEF LagoZ LTR* on November 25, 1999). Table A compares the G+C nucleotides content and the observed/expected ratio (O/E) in CpG dinucleotides for the three genes. According to well known criteria, the LacZ gene corresponds to a very CpG rich island since its O/E is superior to 0.6 (Larsen et al. 1992). The LagZ gene corresponds to a sequence poor in CpG with a O/E closed to the ratio observed in the genome, HORS the CpG rich island. The LagoZ gene corresponds to a sequence entirely devoid of CpG. Such a situation is never found in the genome. The C+G content of the LagZ and LagoZ genes stays close to 50%.

**TABLE A: CpG content of LacZ, LagZ and LagoZ genes**

| **Genes** | **Nbr of CpG** | **% of CpG** | **% of G+C** | **O/E (G+C)** |
|---|---|---|---|---|
| **LacZ** | 291 | 9,24 | 55,80 | 1,18 |
| **LagZ** | 52 | 1,65 | 49,30 | 0,27 |
| **LagoZ** | 2 | 0,06 | 48,20 | 0,01 |

| | | | | |
|---|---|---|---|---|
| Nbr of CpG = number of CpG in each sequence. O/E (G+C) = number of observed CpG over the expected number of CpG | | | | |

LacZ, LagZ and LagoZ genes were combined to various promoters in order to test whether LagZ and LagoZ genes still posses the capacity of being transcribed and translated although the 239 modifications introduced into the LagZ gene and the 291 modifications introduced into the LagoZ gene. Some of these promoters are known to control the expression genes devoid of tissular specificity such as the promoter of the α sub-unit of the elongation factor 1 of the translation (E1Fα) (Uetsuki et al, 1989) and the hypoxantine phosphoribosyl-transferase promoter (HPRT). The results presented herein concern the E1FαLacZ, E1FαLagZ and E1FαLagoZ constructions. These constructions were injected into mouse eggs male pronucleus and into the nucleus of one of the two embryo blastocysts at the 2-cell stage. For the E1FαLagoZ gene, two types of molecules were tested: the whole plasmid which still contained external sequences of the CpG rich gene and a fragment wherein these sequences were deleted. No differences were seen between both experiments. In every case, an expression was observed and the labeling corresponded to the labeling of an enzyme having a nucleus addressing sequence. Some of the eggs which have remained blocked at the 1-cell stage were positive. No quantitative difference was noted in the β-galactosidase enzymatic activity (Table B). As a result, none of the introduced or fortuitous mutations did significantly affect the β-galactosidase activity, nor the nuclear localization conferred by the nuclear nls addressing sequence (Bonnerot et al, 1987)

**TABLE B: Assay of the β-galactosidase activity of E1FαLacZ, E1FαLagZ and E1FαLagoZ genes**

| **Genes** | **Nbr of embryos** | **Activity** **(U** β**-gal x 10⁻⁵)** |
|---|---|---|
| **LacZ** | 12 (32) | 5,6 (± 1,3) |
| **LagZ** | 17 (37) | 5,7 (± 5,3) |
| **LagoZ** | 21 (32) | 7,1 (± 9,4) |

| | | |
|---|---|---|
| Does represent an estimation of the β-galactosidase activity following the microinjection (5000 copies) of each one of the genes into 1 cell stage mouse embryos (transitory assays). The activity for each egg was measured using the "MUG" technique. (_) = number of embryos tested. | | |

### 4.0 Discussion

This study has shown that the total absence of CpG in the coding part of a 3000 pb gene (a situation which has no equivalency in the genome of mammals) has no effect, on the short term, on the expression of this gene. Indeed, the expression levels of the LagoZ gene were similar (if not higher) than the LacZ and LagZ genes. This applies to genes placed into a nuclear environment of a 1-cell stage male pronucleus as well as in the 2-cell stage zygotic nuclear environment. These two stages correspond to two different stages of the transcriptional machinery of the genome, which are before and after the acquisition from the embryo, of the distance activation competency (Forlani and Nicolas, 1996). The LagZ and LagoZ expressions thus demonstrate that the modifications introduced to the primary DNA sequence did not create any sites which are recognized by suppressor present at these stages. Furthermore, the similar quantitative level of β-galactosidase activity of the three genes demonstrates that no pattern responsible for the splicing or any other modifications to the RNA properties or the enzyme has been created during the mutagenesis.

The results presented herein were obtained by combining the LacZ, LagZ and LagoZ genes to the promoter's area (promoter, first exon and first intron) of the α sub-unit of the elongation factor 1 of the translation (HSEF1α). Since this gene is one of the genes which is transcribed and translated at high levels in the cells, a normal expression of the three genes was anticipated, even into non-terminal differentiated cells. The next step was thus to test if similar levels of expression could be obtained into cells at a different stage of development, particularly into somatic cells wherein genome methylation reaches maximum levels.

### EXAMPLE 2:

### ESTABLISHMENT AND RELIEF OF CpG-DEPENDENT TRANSGENE SILENCING DURING GERM LINE PASSAGE AND MOUSE DEVELOPMENT

### 1.0 Introduction

Numerous studies have demonstrated a repressive effect of methylated CpG (mCpG) on gene expression in vertebrate differenciated cells (Hsieh, 1994; Kudo, 1998; Goto and Monk, 1998; Jones et al., 1998; Collas, 1998) This repressive effect of mCpG is equally efficient when either the promotor part or only the structural part of the gene are methylated (Trasler et al., 1990) (Komura et al., 1995; Nan et al., 1997; Singal et al., 1997). However, the density of mCpG must reach a treshhold value to induce repression (Hsieh, 1994; Komura et al., 1995; Kass et al., 1997; Nan et al., 1997; Goto et al., 1998). Methylated CpGs do not need to be clustered as a dense island to prevent expression. When dispersed on several kilobases, mCpGs are still efficient (Boyes and Bird, 1991; Hsieh, 1994; Nan et al., 1997; Goto et al., 1998). The repressive effects of DNA methylation are probably mediated indirectly since these effects are relieved in trans by methylated oligonucleotides *in vitro* as *in vivo* (Boyes and Bird, 1991; Nan et al., 1997) and since the repression appears only after a delay of several hours in transient expression systems (Buschhausen et al., 1987; Kass et al., 1997).

Methyl DNA binding proteins (MBD) are likely mediators for the biological effect of mCpG (Hendrich and Bird, 1998; Kudo, 1998). MBDs act as part of multiprotein complexes. For instance, MeCP2 associates with methylated DNA of the somatic genome (Nan et al., 1996) as a complex including Sin3A and histone deacetylases HDAC1 and HDAC2 (Nan et al., 1998; Jones at al., 1998). So, it is conceivable that the MCpG-dependent repression is due, at least in part, to the remodelling of chromatin structure through histone deacetylation (Jones et al., 1998). In addition, MeCP2 can inhibit gene expression at a distance from promotor (Nan et al., 1997). Nevertheless, the in vivo implication of this complex in gene expression remains hypothetical since only artificial systems have so far been analysed where MeCP2 is directed towards DNA through a GAL4 DNA binding domain and not by its proper DNA binding domain (Nan et al., 1998). That this system may indeed operate in vivo is suggested by observations of genes repressed by methylation which gain expression after treatment with inhibitors of deacetylases (Boyes and Bird, 1992; Hsieh, 1994; Singal et al., 1997; Jones et al., 1998; Nan et al., 1998). MBD1, another MBD protein is also included in a large complex of 800 kD (MeCP1) (Boyes and Bird, 1992), the components of which are not yet determined. All these features suggest that mCpG and the associated MBD proteins constitute a general non-specific repressive system of gene transcription in differentiated cells.

The pattern of methylation of the genome and of genes is maintained in dividing somatic cells, as best exemplified by the cases of hypomethylated or hypermethylated DNA introduced into cells (Hsieh, 1994; Howell et al., 1998; Kudo, 1998). In contrast, this pattern is dynamic during development and gametogenesis (Monk et al., 1987; Sanford et al., 1987; Trasler et al., 1990; Ariel et al., 1991; Kafri et al., 1992; Ghazi et al., 1992; Warnecke and Clark, 1999; Martin et al., 1999). The global methylation of genome is maximal in the embryo at gastrulation and minimal in cells of the blastocyst. Sperm and oocytes present an intermediate level of methylation and a demethylation is observed during the first cleavages of the embryo (Monk et al., 1987; Sanford et al., 1987; Rougier et al., 1998). The DNA of male and female germ cells at 12.5-14.5 dpc is hypomethylated (Monk et ai., 1987).

The methylation pattern of some endogenous genes also follows this general sheme. In particular, nearly systematically, an hypomethylation is observed at blastocyst stage, a stronger methylation is observed at the following stages and in somatic tissues and an hypomethylation at the beginning of male and female gametogenesis (Kafri et al., 1992; Warnecke and Clark, 1999, Martin et al., 1999). However, in addition to this general pattern, numerous variations have been observed at specific genes which illustrate the existence of complex methylation and demethylation processes particularly during male gametogenesis and in somatic tissues (Trasler et al., 1990, Kafri et al., 1992; Groudine and Conkin, 1985; Warnecke and Clark, 1999). In some cases, the methylation level seems to be correlated with expression (Trasler et al., 1990; Zhang et al., 1998; Goto et al., 1998; Salvatore et al., 1998; Cameron et al., 1999), but in others, such a correlation is not found (Weng et al., 1995; Zhang et al., 1998; Warnecke and Clark, 1999). In at least one case, the methylation of promotor seems unchanged the gene being expressed or not (Warnecke and Clark, 1999). In another case, the global density rather than specific sites distinguishes expressed to not expressed alleles, which suggests that the functionning of a gene does not necessarily require demethylation at particular sites (Salvatore et al., 1998). Thus, the concept of tissue-specific gene expression being controlled by a selective demethylation is not completely verified (Trasler et al., 1990; Walsh and Bestor. 1999). Recent studies, still incomplete, of the methylation of the DNA of endogenous genes by bisulfite sequencing which allows the detection of the methylation state of all CpGs of a gene from a single cell confirm these data and reveal an amazing heterogeneity of pattern of methylation of genes in different cells for any stage analysed (Salvatore et al., 1998; Warnecke and Clark, 1999: Cameron et al., 1999).

The repressive indirect effect of Methylation and the dynamic pattern of methylation during development, raise a potential paradox which, if resolved, would have major consequences for our understanding of the evolution of the sequence of ubiquitous and tissue-specific genes in vertebrates. Indeed, if at a certain density of methyl-CpG, MBD proteins act as an indirect general repressive system on gene expression and if the methyl-CpG density of genes strongly varies at some stages, then genes expression should be sensitive to these variations. Otherwise, to preserve their spatio-temporal expression tissue-specific or ubiquitous, the sequence of genes would have to differentially adapt to these conditions.

It is crucial to test *in vivo* if, indeed, gene expression is sensitive to the fluctuations of the methylation during development, but, so far, no system has allowed this hypothesis to be tested.

We describe here such a system and the first results of the testing of this hypothesis. The experimental system used compares in transgenic mice the expression of a LacZ reporter gene for which the density of CpG sequence is higher (8.6 %, 302 sites for 3.5 kb) than that of endogenous genes and the same reporter gene which CpG level has been lowered by directed mutagenesis to a percentage close to that of endogenous genes (2.2%, 78 sites for 3.5 kb). To be able to explore different stages during development and gametogenesis, these two genes have been combined to a strong promotor of an ubiquitous gene, the promotor of the gene coding for the human translation elongation factor, EF1U (Uetsuki et al., 1989). We have also studied the expression of the CpG rich reporter gene controled by a weak ubiquitous promotor, the promotor of the gene for the human hypoxanthine phosphoribosyl transferase, when associated or not with the minilocus control region of β-globin (Talbot et al. 1989). Results show that at periods of genome hypomethylation, both CpG-poor and CpG-rich reporter genes associated to ubiquitous promotors are expressed whereas only the CpG poor reporter is expressed at periods of genome hypermethylation in embryonic and somatic cells after implantation. Moreover, we show that CpG-rich transgenes are repressed at several stages during male and female gametogenesis and, depending on the parental origin, in the eariy embryo where a strong expression is observed for only CpG-poor transgenes. This is the first proof that gene expression in vivo is regulated by the fluctuations of a CpG-dependent negative control system. Finally, this repression of CpG-rich transgenes can be completely reversed by tissue specific trans-activating factors in specialized cells and relieved by treatment with inhibitors of histone deacetylases in preimplantation embryos. This suggest that several of the CpG-dependent repressive effects observed during development and gametogenesis are mediated by histone deacetylation of chromatin.

### 2.0 Materials and methods

### 2.1 DNA Inserts

Constructs of the HPRTnlsLacZ and HPRTnlsLacZDCR inserts was previously described in (Bonnerot et al., 1990) and (Bonnerot and Nicolas, 1993a). They contain the promotor of the human hypoxanthine phosphoribosyl transferase (HPRT) gene that' drives expression of a nuclear targeted β-galactosidase (nlsLacZ). HPRTnIsLacZDCR contains the four Dnasel hypersensitive sites of the human LCR β-globin gene (Talbot et al., 1989). The 7.9 kb EFnlsLacZ insert was isolated from the plasmid, pBSEFnlsLacZdenn, as a Xhol-Notl-Notl fragment (partial digestion for Xhoi). pBSEFnlsLacZdenh was derived from pEF321-CAT kindly provided by Kim D.W. (Kim et al., 1990). The 2.3kb HindIII-ScaI fragment of pEF321-CAT containing the (+1) to (+1561) portion of the human EF1α gene (promotor, exon 1 and intron 1), plus 730 bp of 5' untranslated sequence (Uetsuki et al., 1989) was ligated (after klenow fill-in) to a 9.5 kb Sall fragment of pBSGAdLTRnlsLacZ (Bonnerot et al., unpublished), containing the nlsLacZ reporter gene and the polyadenylation signal of Moloney murine leukemia virus on a pBluescript plasmid backbone.

### 2.2 Mutagenesis of the LacZ gene and generation of the EFLagZ insert

The CpG content of LacZ was lowered from 9.2 % to 2.2 % , a percentage close to that of the mammalian genome by mutagenesis. A Polymerase Chain Reaction (PCR) technique was used, in which the mutagenic oligonucleotide primers were designed to preserve integrity of the amino acid sequence of the β-galactosidase reporter protein. The DNA sequence of the mutated LacZ was verified by sequencing.

For construction of pBEFnlsLacZdenh, nlsLacZ was replaced by nlsLagZ after ligation of the 3.5 kb Avrll-BamHl LagZ fragment of pPytknlsLagZ to the 3.5 kb AvrII-BamHI fragment of pBEFnlsLacZδenh, lacking nlsLacZ. The resulting plasmid pBEFnlsLagZδenh was digested by Xhol-Notl (XhoI partial) to obtain the 7.9 kb insert EFnlsLagZ.

### 2.3 Transgenesis

Plasmids were digested to remove vector DNA sequences and inserts were purified on glass beads. Transgenic mice were generated as described in Forlani et al. (Forlani et al., 1998).

### 2.4 Recovery of embryos and cryosectioning

Preimplantation embryos were recovered from crosses between (B6D2) F1 females or males and transgenic males or females, respectively, as described in (Forlani et al., 1998; Vernet et al., 1993). Ovaries and testes were dissected from embryos at different ages according to protocols described in (Hogan et al., 1986) Embryonic testes were identified by the presence of seminal cords. After dissection organs, X-gal staining and cryosectioning were performed as described in (Bonnerot and Nicolas, 1993).

### 2.5 Qualitative and quantitative analysis of β-galactosidase activity

For analysis of transgene expression in preimplantation embryos, freshly harvested or cultured embryos were recovered at the appropriate times and immediately analysed by X-gal staining overnight at 300° C (Vemet et al., 1993). Embryonic and adult organs were stained for two days at 300° C and cryosections overnight at 300° C (Bonnerot et al., 1990). In some experiments, quantification of β-galactosidase activity was used to screen adult males according to their transgene expression in testis. A single testis was surgically removed such that transgenic males could be subsequently mated with (B6D2) F1 females to generate preimplantation embryos.

The removed testis was cut into two parts. The first half was fixed in 4 % PFA and X-gal stained. The second half was used to recover proteins and to measure β-gal activity using an assay that measures cleavage of the fluorogenic substrate, 4-methylumbelliferyl β-D galactoside (Forlani and Nicolas 1996).

### 3.0 Results

For this study, the bacterial LacZ reporter gene has been used, because it is a CpG rich region according to criteria defined in (Larsen et al., 1992) with a G+C content above 50% (%(G+C)54.4%) and a ratio observed of observed versus expected CpG (O/E) above 0.6 (O/E=1.17), that are potential targets for methyl CpG binding proteins and their associated partners (Hendrich and Bird, 1998) (Jones et al., 1998). For comparison, a modified CpG-poor LacZ gene has been constructed, from which 224 CpG sites were replaced by directed mutagenesis to achieve characteristic of non CpG rich sequence with a %(G+C) of 48.9% and a O/E of 0.37. This new reporter has been called LagZ and, as with LacZ, was used as a reporter in association with a nuciear localization signal in order to readily identify expression in all tissues and at all stages during embryogenesis (Bonnerot et al., 1987). These two sequences have been fused to a very strong promotor from the gene encoding the human translation elongation factor EF1α, whose expression is ubiquitous in the mouse (Fig. 2) (Hanaoka et al., 1991). To examine a transgene with a different ratio between cis-activating and cis-repressive elements, nls LacZ was fused to a weaker, though also ubiquitous, promotor from the human hypoxanthine phosphorybosyl transferase gene (HPRT) (Fig. 2) (Bonnerot et al., 1990). Finally, the HPRTLacZ transgene was combined with the mini-locus control region of the β-globin gene to determine the effect of this strong activating element on a potentially repressed structure in a specific somatic lineage (Bonnerot and Nicolas, 1992).

### 3.1 Similar levels of transient expression of the CpG-rich (EFLacZ) and CpG-poor (EFLagZ) transgenes after microinjection into fertilized egg

To test whether the CpG content or other sequence differences between the LacZ and LagZ genes influenced their expression in the absence of methylation, EFLacZ and EFLagZ DNA constructs (depleted of plasmidic sequences) were microinjected as inserts into the male pronucleus of fertilized eggs at 20-22 hphCG. Expression was then analysed by X-gal staining, once eggs had cleaved (46-48 hphCG). Both inserts were expressed in about half of injected eggs (eleven eggs were microinjected for each insert) and their level of expression was comparable (data not shown). Therefore the EF1α gene promotor is capable of driving expression of both the reporter genes in the cleaved embryo, and all trans elements required for expression are present at this stage. Sequence differences, including their CpG content, do not change the expression level.

However, an analysis of transient expression of inserts does not reveal how expression evolves during development, nor the influence of transgene passage through gametogenesis. To study these questiona, transgenic mice containing EFLagZ (EFLagZ1 to 3) and EFLacZ (EFLacZ1 to 4) as stable transgenes were generated. In all seven lines, the transgene was integrated in an autosome.

### 3.2 Expression of EFLacZ and EFLagZ correlates with a variation in the global methylation of the genome

Global methylation of the genome is minimal in blastocysts and maximal in the cells of implanted embryos (Monk et al., 1987) (Kafri et al., 1992) (Sandford et al., 1987). To check whether LagZ and LacZ are sensitive to these global changes, we first analysed the expression pattern of the transgenic lines at these two stages.

At the blastocyst stage, the three EFLagZ and the four EFLacZ lines all expressed the transgene, demonstrating that whatever the CpG content, the reporter gene is in a permissive state for expression (data not shown). In addition, the parental origin of the transgene did not affect the expression, except for the EFLacZ1 line in which the maternally inhented transgene was not expressed.

After implantation, the expression pattern of the EFLagZ lines was clearly different from that of the EFLacZ lines. At 13.5 dpc, none of the EFLacZ lines expressed the transgene, neither in extraembryonic tissues such as the yolk sac nor in somatic tissues. In contrast, we observed a constant level of transgene expression for all EFLagZ lines in the yolk sac and in embryos (data not shown). However, in both cases, the expression was variegated, with only a fraction of the cells expressing LagZ. For instance, in the yolk sac, the labelling was distributed in clusters of cells which strongly suggests a clonal transmission of the permissive state for expression, since growth in this tissue is known to be coherent (Gardner and Lawrence, 1985). In addition, transgene expression was still detected in EFLagZ adults in a variety of tissues (data not shown). In contrast, in EFLacZ mice, expression was never observed in adult tissues. Therefore, a differential expression corresponding to the CpG content of the transgene appears after implantation and is subsequently maintained throughout development and into aduithood. From these results we conclude that, even in combination with a strong ubiquitously active promotor, a high CpG content leads to complete gene inactivity whereas a low CpG content leads to gene activity in at least a fraction of cells. Because the comparison between the expression of these two transgenes gave valuable information about the implication of CpG density in gene inactivity, we next analysed the expression of EFLacZ and EFLagZ during gametogenesis and early development, since information concerning the methylation status of these developmental stages is not as clear as in blastocysts and somatic cells.

### 3.3 Differential expression of EFLagZ and EFLacZ transgenes during oogenesis

Global genome methylation fluctuates during oogenesis. The maternal genome is demethylated in primordial germ cells, then further demethylated during Preleptotene-Leptotene-Zygotene (P-L-Z) and is finally methylated at an unknown stage, such that a moderate level of methylation is attained at the terminal stages of oogenesis (Monk et al., 1987). In contrast, analysis of the methylation status of individual transgenes during oogenesis has shown that, in general, maternal transgenes remain hypermethylated (Chaillet, 1994).

Transgene expression in the EFLagZ and EFLacZ transgenic lines during oogenesis was examined in female gonads from E12.5 embryos, in which oocytes were at the P-L-Z stages of prophase I and also in the adult, where oocytes were blocked at metaphase II (Fig. 3A). Ovulated oocytes were also analysed and these correspond to the transcriptionally inactive gametes (Schultz, 1986).

In the EFLagZ lines, the transgene is expressed in virtually all female germ cells, as early as the preleptotene stage of prophase I (13.5 dpc). A continuous β-gal activity was also detected in all subsequent stages: at the pachytene stage (15.5-16.5 dpc and birth), during the growth phase at diplotene (starting at 8 dpp) and at metaphase II in the adult gonad (data not shown).
As expected, β-gal activity was not detected in the transcriptionally silent ovulated oocytes (Fig. 3B). The identical and continuous expression of the EFLagZ transgene observed for different lines during oogenesis confirms that the pattern of expression is independent to the integration site (transgene-dependent) and demonstrates that trans activators for the EF1α gene promotor are constitutively active in oocytes until the transcriptional arrest that characterizes the terminal stage of oogenesis. The EFLagZ3 line also expressed the transgene during oogenesis but only transiently at 16.5 dpc (Fig. 3B); this effect is probably due to a position effect of the transgene (see below).

In contrast, none of the CpG-rich EFLacZ lines continuously expressed the transgene during oogenesis (not shown). In the ELFacZ4 line, the transgene was never expressed and in EFLacZ1, EFLacZ2 and EFLacZ3, the transgene was only transiently expressed at the pachytene stage of prophase I (16.5 dpc for EFLacZ1 and 2 lines) and at birth (EFLacZ3) (Fig. 3B). In the adult ovary, only the EFLacZ2 line expressed the transgene and only in a few gametes at the beginning of growth phase (not shown). This absence of expression at preleptotene, leptotene, zygotene and then at the diplotene stage is, therefore, transgene dependent. The expression of the EFLagZ transgene at the corresponding stages indicates that the absence of expression is not due to a lack of trans-activators for the EF1α gene promotor.

Taken together, these results reveal that the pachytene stage of oogenesis is particular, because both EFLagZ and EFLacZ transgenes are expressed. This period of expression during pachytene is flanked by two periods of repression which are CpG-dependent; notably EFLacZ transgenes are silenced beginning at the diplotene stage in growing oocytes.

### 3.4 Expression of EFLagZ and EFLacZ transgenes during spermatogenesis

Global methylation studies during spermatogenesis indicate that the paternal genome is demethylated in primordial germ cells and is then found methylated in sperm at an intermediate level (Monk et al., 1987). Nearly nothing is known concerning the evolution of global methylation between these two stages of male gametogenesis. The analysis of methylation for several genes containing CpG at specific positions has shown that both demethylation and methylation events can occur in meïotic cells (Trasler et al., 1990; Ariel et al. 1991; Kafri et al., 1992).

In the male embryo, gonocytes are dividing from 12.5 to 16.5 dpc, then arrest in G1 (Vergouwen et al., 1991). At birth, the first spermatogenic wave begins with appearance of type A spermatogonies. At 8 dpp type B spermatogonies appear and two days later, primary spermatocytes (the preleptotene, leptotene and zygotene stages) arising from the division of type B spermatogonies (Kluin and de Rooij, 1981). Finally, primary spermatocytes at the pachytene stage appear, along with post-meïotic round spermatids at 14 and 20 dpp respectively. The terminal differentiation stages, involving generation of elongated spermatids and spermatozoa, occur during the following 15 days (Fig. 3A).

Analysis of the first wave of spermatogenesis in the ELFagZ lines indicates that their transgene is expressed continuously in male germ cells. β-gal activity was detected very early, in gonocytes (12.5-13.5 dpc), as well as in type A spermatogonies at birth, and type B spermatogonies at 8 dpp. This pattern was maintained at all stages in adult testis, for which all stages of spermatogenesis were examined including elongated spermatids (data not shown).

Similarly, all ELFacZ lines showed β-gal activity in male germ cells (not shown); however the β-gal activity was first detected at birth when type A spermatogonies appeared, as there was no detectable activity in gonocytes. Moreover, the number of β-gal+ type A spermatogonies in ELFacZ lines was lower than in ELFagZ lines. From birth to 8 dpp, the number of ELFagZ ∃-gal+ germ cells, which represent type A and B spermatogonies, increased. In adult testis, β-gal activity was observed in type A spermatogonies up to the round spermatid stage (not shown). The identical and continuous expression of ELFagZ transgene observed for all EFLagZ lines during spermatogenesis (Fig. 3B) confirms that the pattern of expression is transgene-dependent and demonstrate that the trans activators for the EF1α gene promotor are constitutively active in male gametes during all spermatogenesis.

In summary, prior to spermatogenesis, the expression of the EFLagZ gene begins shortly after the transition period between primordial germ cells and gonocytes, at E13.5, and the expression of EFLacZ is delayed until the first appearance of type A spermatogonies. After this differential timing in activation, expression of both transgenes is detected until the transcriptional arrest at the round spermatid stage. These data suggest that a non-permissive state for expression exists in male gonocytes in relation to the high CpG content of transgenes and that a favourable condition later appears in type A spermatogonie cells, which relieves this repressive state.

### 3.5 The Sex-dependent transgene expression during gametogenesis persists in the zygotic nucleus before the morula stage

There is a differential expression of the EFLacZ transgene in male and female germ cells, persisting until the transcriptional arrest in both types of mature gametes. During gametogenesis, a sex-dependent expression of the EFLacZ transgene is mediated by repression of maternal transgene expression at the diplotene stage in relation to its high CpG content. To determine if this sex-dependent expression of the EFLacZ transgene is maintained in the embryo after fertilization, transgene expression in EFLagZ and EFLacZ mice was analysed by X-gal staining of embryos (data not shown). To study the expression of transgene of paternal and maternal origin, embryos were obtained from the progeny of both male and female transgenics crossed with B6D2 F1 animals.

In all EFLagZ lines, the transgene was expressed independently from its parental origin as early as the 2 or 4-cell stage until the blastocyst stage. In EFLacZ lines, the transgene was expressed from the 4-cell stage to the blastocyst stage but only when it was transmitted by a male. In contrast, when the EFLacZ transgene derived from a female, its expression was always detected later and not before the morula stage. Therefore, a parental origin-dependent expression, also related to its high CpG content, characterizes the EFLacZ transgene during the first enmbryonic cleavages of embryo. This differential expression can be compared to previous observations made during gametogenesis. Strikingly, for both transgenes, the expression in cleavage-stage embryos is reminiscent of the expression observed in germ cells: for the paternal and maternal EFLagZ or the paternal EFLacZ transgenes, which are expressed during most of gametogenesis, an early expression is detected during the first cleavages after fertilization (2- and 4-cell embryos); and the maternal EFLacZ transgene, which is not expressed during most oocytic stages, is found expressed after fertilization at later stages (i.e. morula-blastocysts). These results strongly suggest that the permissive or non-permissive transcriptional state of transgenes in differentiating gametes is maintained during the first cleavages of the embryo.

### 3.6 Persistence of the gametic transcriptional permissivity in the preimplantation embryo

Our results suggest that the regulation exerted on EFLacZ and EFLagZ transgenes in the early embryo is previously determined during gametogenesis. The following observations argue for this gameto-zygotic continuity. In some EFLacZ lines, in particular EFLacZ1, a variegated expression was observed in the germ cells contained in the adult seminal tube from one male to another in the same line (Fig. 3C), adut quantitative expression). Therefore, during gametogenesis, the transition between gonocytes and spermatogonies is not followed by relief of a non-permissive transcriptional state in all germ cells. If we postulate that a gameto-zygotic continuity indeed exists, then a correlation should be observed between the level of transgene expression in the adult testis of a male and the proportion of β-gal+ preimplantation embryos that have inherited their transgene from this same male. This comparison has been made using two EFLacZ1 males selected on the basis of the β-gal activity measured in one of their surgically removed testis. EFLacZ1 males expressing a high or low β-gal activity were crossed with non transgenic females to generate 4-cell embryos (data not shown). The transgenic male with a very low β-gal activity in germ cells generated β-gal embryos only while the one with a high β-gal activity in germ cells generated β-gal+ embryos. These results establish a correlation between the transcriptional state of the EFLacZ transgene in male gametes and that of the preimplantation embryos, supporting the concept of a gameto-zygotic continuity of this transcriptional state.

### 3.7 The morula-blastocyst period: A general relief from all gametic repressive states

We have already reported herein above that the EFLacZ transgenes are expressed at the blastocyst stage. Because maternally transmitted transgenes are repressed during the first cleavages, we have investigated in more details at which stage this sex-dependent repression is released. At the morula stage, a certain fraction of the embryos carrying the maternal transgene were already β-gal+ and this fraction increased further at the blastocyst stage. Release from the repression of the maternal transgene begins at the morula stage and seems to be progressive (not shown).

We have also noticed that several EFLacZ lines and one EFLagZ line (EFLagZ3) were characterized by a variegated expression of their transgene during spermatogenesis. β-gal+ germ cells were arranged in clusters along the seminal cord and the overall β-gal activity (MUG) was low (Fig. 3B). Therefore, only a fraction of gonocytes (EFLagZ3) and type A and B spermatogonies (EFLacZ lines) were relieved of the non-permissive state for transgene expression. The most obvious example of this was seen for EFLacZ4. Strikingly, in this line, the paternally transmitted transgene was only active at the morula stage (not shown). Since the morula stage is also the period at which repression of maternal transgenes is relieved, the morula-blastocyst stage appears to correspond to a developmental period when all gametic repressions, applied to both male and female EFLacZ transgenes are released.

### 3.8 A similar regulated expression for the HPRT promotor

To test whether regulatory mechanisms described for EFLacZ were specific for this promotor, other promotors were fused with the LacZ gene and used to generate transgenic mice (Fig. 2). The weak promotor of the ubiquitously expressed human hypoxanthine phosphorybosyl transferase (HPRT) gene was used for these studies and a construct combining the HPRT promotor with hematopoïetic specific enhancers derived from the β-globin locus control region (HPRT-DCR) was also analyzed. Two transgenic lines containing the HPRT insert (HPRTLacZ1 and HPRTLacZ3 lines) were analyzed, along with seven lines containing the HPRT-DCR insert (DCR1 to 7 lines). The transgene expression patterns during gametogenesis and the first cleavages of embryo were examined in the same way as for the EFLagZ and EFLacZ lines described above. First, as previously reported, none of the HPRTLacZ and HPRTLacZDCR lines ubiquitously expressed the transgene in postimplantation embryo, which confirms a general repression of the transgene in somatic cells (data not shown) (Bonnerot et al., 1990; Bonnerot and Nicolas, 1993a).

Second, the male germ cells in all HPRTLacZ lines and six of the seven DCR lines expressed the transgene, at least in the pachytene spermatocytes (Table 2A). None of the DCR lines expressed their transgene in gonocytes. Rather expression began at different times according to the line: a birth for DCR1 and DCR6, at 8 dpp for DCR4 and 7 and at 10 dpp for DCR2 and DCR3. In adult testis, expression was also readily detected at the pachytene stage and at all stages up to the development of round. spermatids. However, we observed variations in the staining intensity from line to line. In particular, the staining in HPRTLacZ mice was lower than in DCR mice (data not shown). Quantitative analysis of the β-galactosidase activity in adult testis confirmed this result (Table 1A).

Third, in female germ cells, a transient transgene expression was detected between 12.5 dpc and 2.5 dpp during the pachytene stage in five DCR lines (Table 2B). For all DCR lines, this period of expression was followed by a period of repression starting at the diplotene stage and continuing up to the full grown stage of the oocyte in the adult ovaries. Therefore, together with the observations made during spermatogenesis, these date indicate that tne sex-dependent expression observed during gametogenesis for the EFLacZ transgene also occurs when the CpG-rich LacZ gene is controlled by the HPRT promotor.

To determine whether the sex-dependent gametic expression of the HPRTLacZ and DCR transgenes is correlated with a parental effect in the cleavage stage embryo (as for EFLacZ lines), expression of paternal and maternal transgenes was tested. Probably because of the weakness of the HPRT promotor, LacZ expression was only detected by X-gal staining in aphidicolin arrested eggs, for which the signal is amplified (see Material and Methods for a more detailed description of this technique).

Fertilized eggs recovered at 24 hphCG were stained 24 hours later, at a time when control embryos reached the late 2-cell stage (Table 2). All lines that expressed the transgene during spermatogenesis also expressed the transgene in arrested 1-cell embryos. Strikingly, none of these lines expressed the maternally transmitted transgene and this parental effect was still observed when embryos were cultured at the 2-cell and 4-cell stage from the DCR6 and DCR7 lines. Therefore, the sex-dependent transgene expression persists through several cleavages after fertilization.

The idea of a gameto-zygotic continuity for the transcriptional state was tested in HPRTLacZ and DCR lines by comparing expression in preimplantation embryos of transgenes inherited from two males of the same line (DCR4, DCR7 and HPRTLacZ1), selected for differences in their β-gal activity in adult germ cells. In all cases, the transgene transmitted by males with a high β-gal activity in testis was also expressed in cleavage stages embryos; whereas embryos derived from males showing a low β-gal activity in testis did not express the transgene (data not shown).

Finally, we studied expression of LacZ transgenes containing tissue-specific promotors, Hoxb-7 (Kress et al., 1990) or AchRα (Klarsfeld et al., 1991). However, expression of the paternal transgene was not detected in germ cells of the adult testis nor in 2-cell embryo blocked by aphidicolin (data not shown). In contrast, the tissue-specific expression of these two promotors was, as expected, observed in post-implantation embryos (Kress et al., 1990; Klarsfeld et al., 1991).

Taken together, these results demonstrate that the regulation described for the EFLacZ transgene in somatic cells, during gametogenesis and at first cleavages after fertilization (parental differential expression, gameto-zygotic continuity), are not specific to the EF1α promotor, but also applies to the association of a CpG-rich LacZ with the weaker HPRT promotor. However, the LacZ gene needs to be combined with promotor sequences of a ubiquituous gene in order to be expressed in germ cells and the embryo. The minimal promotor sequences (TATA and GAAT box) contained in the tissue-specific Hoxb-7 and AchRα promotors seem unable to drive a detectable level of expression in these cells.

### 3.9 Repression of the maternal and paternal LacZ transgenes in embryos before the morula stage is mediated by histone deacetylase complexes

It is becoming more and more evident that at least part of the transcriptional repression dependent on methylated CpG islands is mediated by histone deacetylation. Indeed, the MeCP2/Sin3A/histone deacetylase complex has been shown to bind to methyl CpG (Nan et al., 1998) (Jones et al., 1998) and a large fraction of the deacetylases of the cells are complexed with MeCP2 (Bestor, 1998). To test whether this mechanism could be responsible for the non-permissive transcriptional state established during gametogenesis and inherited by the embryo, cleavage stage embryos were treated with the deacetylase inhibitors, sodium butyrate (NaB) and the trichostatin A (TSA), two inhibitors of histone deacetyfases (Yoshida et al., 1995).

LacZ transgenes from the DCR6 and DCR7 were studied since the transgene of both parental origin is expressed in a small number of 2-cell embryos or no. In both cases, a release from repression was obtained in embryos treated with either NaB or TSA (Fig. 4A). This strongly suggests that the mechanism of repression of the maternal LacZ transgene is mediated by histone deacetylases at the chromatin level. Since we have shown that this repression is also related to the high CpG content in LacZ, it may imply that histone deacetylases act on methylated DNA.

The effect of NaB was also tested on the repressed paternal transgenes which characterize certain transgenic lines. For instance, in the DCR3, DCR1 and DCR5 lines, expression of the transgene was repressed in 82, 97 and 100% of arrested 1-cell embryos, respectively (Table 2). In all three lines, relief from repression was observed in a fraction of the NaB treated embryos (Fig. 4B) and seemed to be related to the percentage of β-gal+ untreated embryos: the greater the β-gal+ percentage (18%. 3% and 0% for DCR3, DCR1 and DCR5, respectively) in untreated embryos the greater the proportion of β-gal+ embryos after NaB treatment (100%, 70% and 10% respectively). Therefore, as for the maternal transgenes, these data suggest that paternal transgenes may be locally repressed at the chromatin level by histone deacetylases in some embryos. Moreover, the correlation between the percentage of β-gal+ embryos before and after NaB treatment suggests that quantitative and not qualitative differences in the level of inhibition account for the observed differences between transgenic lines and between transgenes in the same line. These quantitative differences may result from the relative degree of CpG methylation and may determine the relative dependance on histone deacetylase activity.

### 3.10 Repression of LacZ transgenes in somatic tissues can be relieved by lineage-specific activators

To determine whether the repressive state of HPRTLacZ in embryonic cells can be reversed when a lineage specific activator, LCR, is developmentally switched on, we expanded a previous observation (Bonnerot and Nicolas, 1993a) by examining nucleated erythrocytes for the presence of β-gal activity in the yolk sac at 8.5 and 10.5 dpc and in the fetal liver at 15.5 dpc. All DCR lines expressed the transgene in erythrocytes, including those that exhibited incomplete release from the gametic repressive state during early development (DCR1, DCR3, DCR4 and DCR5, data not shown). In addition, we have already shown that both HPRTLacZ and DCR transgenes are activated by integration site-dependent elements and these elements probably function in an analogous manner to the LCR. Because site-dependent expression involves many cell types, the repressive state clearly can be completely relieved by activators in many, if not all, somatic tissues.

### 4.0 Discussion

This comparison between expression of the LagZ and LacZ transgenes is the first work to demonstrate in vivo the influence of CpG density of the transcribed region of a gene on its expression, to show that variations of the global methylation during development and gametogenesis influence gene expression and to chronicle variations of the repression at specific stages of development. It offers new insigt into: (1) the capacity of the CpG-dependent regulatory systems to induce a non-permissive transcriptional state for genes in vivo, (2) the relief of this state in gametes and embryonic cells, (3) the occurrence of cyclic demethylation at the level of individual genes.

### 4.1 A system to explore CpG-dependent regulatory mechanisms

Because the activity of promotors used in this study depends on ubiquitous transcriptional factors, which remain relatively constant in the cell at all development stages (Kim et al., 1990; Hanaoka et al., 1991), the fluctuations in transgene expression must result primarily from modifications of elements responsible for negative control, such as the mCpG-dependent repressor complexes that modify chromatin structure (Boyes and Bird, 1991; Boyes and Bird, 1992; Nan et al., 1998; Hendnch and Bird, 1998; Jones et al., 1998). Several arguments implicate the CpG-dependent-negative systems, and in particular CpG methylation, in the regulation of most of the variations in transgene expression (summarized in Figure 5) rather than other variations by passing these systems. First, transitions between periods of gene inactivity and expression for the LagZ and LacZ transgenes were never sharply defined but rather spread across several gametic or embryonic stages implying a progressive mechanism rather than a rapid qualitative phenomenon. Second, a remarkable parallel is observed between the CpG-rich LacZ reporter expression pattern and the changes in genomic methylation during gametogenesis and early development. Indeed, the two periods of maximal hypomethylation correspond to the blastocyst and pachytene stages of oogenesis (Monk et al., 1987; Kafri et al., 1992; Rougier et al., 1998; Warnecke and Clark, 1999). At these two stages, our transgenes EFLacZ, EFLagZ and HPRTLacZDCR were expressed in most of the lines. Similarly just after embryo implantation, a period of maximal methylation, we observed the expression of only the CpG-poor transgene (EFLagZ). Third, during the embryo cleavage-stage, a crucial period of transition between a non-permissive and permissive state for LacZ tarnsgenes, inhibitors of histone deacetylases almost completely relieve the repression of maternal transgenes, and that of paternal transgenes still repressed. It demonstrates that both examples of repression result from the deacetylation of chromatin. Taken together with the differential expression of LagZ and LacZ transgenes at these stages, these findings strongly suggest that this deacetylation is CpG-dependent. Therefore, it may involve the MeCP2/Sin3A/HDAC complexes. Another indication that a repressive mCpG system is active in early embryo comes from the observation that methylated genes are repressed at these early stages of development in mice (Goto et al., 1998) adn In Xenopus Laevis (Jones et al., 1998).

### 4. 2 A CpG-dependent repression is active in relation with the richness in CpG content

In *in vitro* systems and in differentiated cells respectively, it has been shown that artificially methylated DNA is indirectly repress by MBD proteins (Nan et al., 1998; Jones et al., 1998) and that this repression is only effective when a certain level of methylation is reached (Hsieh, 1994; Komura et al., 1995; Kass et al., 1997; Nan et al., 1997; Goto et al., 1998). Our results demonstrate that the presence in the transcribed regions of a sequence containing a high density of CpG can create a non-permissive transcriptional state. In cells of the embryo at about 7.5 dpc and in somatic cells, almost all CpGs, except those in CpG islands of promotors, are methylated (Monk et al., 1987; Bird, 1992; Kafri et al., 1992). Therefore, the non-permissive state of LacZ transgenes in embryonic cells just after implantation and later in somatic cells can be attributed to its initiation to a CpG-dependent repressive system. Among the four combinations of sequences tested, only the one containing a strong promotor (E1Fα) and the poor CpG density (LagZ) escapes, although partially, this repression. Therefore, in addition to being a control which demonstrates the implication of CpGs in the regulation of expression, it also shows that even a sequence with a low CpG density can repress. This suggests that *in vivo* the repressive system is determined by a critical threshold of mCpGs.

This leads to the suggestion that it is the global balance between activators and this CpG-dependent repression which controls the activity of a gene. For a gene containing the EF1α promotor, the threshold for inactivation in somatic cells seems to be close to 2% of CpG in the coding region with a %(G+C) of 48.9% and a O/E of 0.37. This is supported by the fact that the coding region of the ubiquitous human EF1α gene replaced by the reporter gene contains 1.3% of CpG with a %(C+G) of 41% and a O/E of 0.29. The use of a reporter gene lacking more CpGs than LagZ and its association with promotors of different strength should allow to define the threshold at which the expression of a gene become insensitive to these negative regulatory effects.

These conclusions lead us to suggest the following hypothesis. As 82% of genes with a broad expression have a CpG poor transcribed region (Larsen et al., 1992), we suspect that their promotors may have a low tolerance to the CpG content and that the sequence of ubiquitous genes may have evolved towards a CpG paucity to counteract the massive and non-discriminatory inhibitory effect induced by the CpG-dependent repressive system.

### 4.3 Capacity of tissue specific trans-activators to relieve the CpG-dependent non permissive state of embryonic cells

The HPRTLacZDCR transgene which combines a relatively weak promotor to a CpG rich sequence is in a non-permissive transcriptional state in embryonic cells after implantation. However, remarkably, this repression is completely relieved by the LCR in embryonic and foetal hematopoïetic lineages, and also, by activator elements at the integration site, which confer to transgenes the position-dependent expression pattern also observed in HPRTLacZ lines (Bonnerot et al., 1990; Bonnerot and Nicolas, 1992). These results indicate that the CpG dependent repressive state does not prevailed over tissue-specific activation. Similarly, it has been shown that enhancers can relieve the inhibition of methylated DNA in in vitro system and in differentiated cells (Boyes and Bird, 1991).

If we follow the idea that gene activity is controlled by the global balance between activators and the CpG-dependent repression acting on chromatin structure, then the relief of repression by the LCR and activators of the non-permissive state of LacZ would be achieved by targeting elements capable of counteracting the action of MBDs complexes. From this point of view, it is remarkable to note that several of the factors associated with RNA polymerase II and several transcription factors are acetylases (Brownell and Allis, 1996; Struhl, 1998). These elements have, therefore, the potential to counteract the deacetylation of histones by the MBD-HDAC complexes and thereby to change chromatin structure.

Then, the artificial combination of an ubiquitous promotor, a CpG rich region and a lineage specific activator mimics remarkably the fundamental properties of a tissue-specific gene. It is notable that as with the LacZ transgenes, nearly all tissue-specific genes also contain a CpG rich sequence in their transcribed region (Larsen et al., 1992). If these CpG islands worked as inhibitory elements in somatic tissues using the process described here for the LacZ gene then the function of transcriptional activators would be to relieve an active repression. As this does not necessitate demethylation of the CpG but rather targeting of elements capable of counteracting the action of MBD proteins (such as acetylases), the apparent paradox of activation of a gene in absence of demethylation would be resolved.

### 4.4 Cycles of methylation/demethylation of the genome during development and gametogenesis.

Is there a developmental control for the establishment of the non-permissive transcriptional state of transgenes? Our results suggest that, at the morula-blastocyst stages, this repression has not yet been established (or is not yet effective on gene expression). Then, prior to cellular differentiation, between blastocyst stage and 7.5 - 10.5 dpc, the specific disappearance of LacZ expression indicates that repression is effective. This inhibition concerns both embryonic and extra-embryonic tissues. It is important to note that since the process is intrinsic to cells, each cell can respond individually. This may explain the observed heterogeneity between cells in EFLagZ embryos. Again these observations are reminiscent of the methylation status of genes and of the genome observed in the embryonic and extra-embryonic tissues (Monk et al., 1987) and of the heterogeneity of the methylation of genes in different cells observed using bisulfite sequencing (Salvatore et al., 1998; Warnecke and Clark, 1999; Cameron et al., 1999).

In both male (gonocytes) and female (Pl-Lp-Zy stages) germ cells, just prior the entry in gametogenesis, the CpG rich transgenes are in a non-permissive state while the CpG poor transgenes are active (Fig 5). This CpG-dependent repression of transgenes is reminiscent of the one established at implantation of the embryo, and suggests a sufficient level of methylation for repression of the LacZ transgenes. However, since the expression of LagZ transgenes is higher in germ cells than in embryonic and somatic cells, the activation/repression balance in germ cells may be inclined more towards a genic activity than towards a repressive state.

In spermatogenesis, LacZ β-gal+ type A spermatogonies appear and their number increases between 0 and 8 dpp. A more detailed study will indicate whether the relief of non-permissiveness is specific to type A spermatogonies or whether it also concerns subsequent stages, especially post-meïotic stages. But clearly, this relief does not occur in preceeding A spermatogonies and particularly in the stem cells (type As spermatogonies). Indeed, if this was the case, the heterogeneity of expression observed during gametogenesis for certain transgenic lines would be erased with aging in males. This heterogeneity is strictly maintained for long periods, as shown by the same expression level in gametes of the same male after a period of six months.

What mechanism relieves the non-permissive transcriptional state in type A spermatogonies? A strong candidate is DNA methylation because the LacZ transgenes whch are still repressed at the entry of male gametogenesis have been shown to be repressed at the 2-cell embryo and are activable by inhibitors of histone deacetylases. It suggests that their CpGs are methylated and that those of the active transgenes are unmethylated (if not, they should be repressed). If this is actually the case, then, a first demethylation of the LacZ gene would occur at the entry of cells in spermatogenesis and later at the morula-blastocyst stage. Several studies indicate that sperm DNA is relatively methylated (less than somatic cells but more than early germ cells) (Monk et al., 1987; Warnecke and Clark, 1999) but other suggest low level of mthylCpG (Trasler et al., 1990).

During oogenesis, at the pachytene stage, both LagZ and LacZ transgenes are active, even though the relief of their non-permissive state begins at different times according to transgenic lines. Since the genome in female germ cells at the pachytene stage is minimally methylated (Monk et al., 1987), it is tempting to attribute this state of activity to the demethylation of CpGs. Later, at the diplotene stage, all LacZ transgenes are again in a non permissive state, which is only relieved at the morula-blastocyst stage. During the first cleavages of the embryo, the relief of the non permissive state of the maternal LacZ transgenes is achieved through the inhibition of histone deacetylases whereas the maternal LagZ transgenes are already active. All these observations suggest that the non permissive state in oocytes is due to methylation of CpGs. Many studies indicate that the DNA of mature oocytes is methylated (Monk et al., 1987; Kafri et al., 1992). Our study suggests that at the level of individual genes, a maximal demethylation occurs in oocytes at the pachytene stage, followed by an active remethylation at the diplotene stage, and finally demethylation of maternal transgenes occurs in the embryo at the morula-blastocyst stage. Although more complex than spermatogenesis, the situation described here for maternal transgenes corresponds again to a cycle of demethylation/methylation.

### 4.5 Biological significance

Our data indicate that CpG rich transgenes are subject to negative control in embryonic and somatic cells and are activated by positive control elements upon cell differentiation. This is compatible with the concept of a global negative control of the genome (Bird, 1995), even for tissue-specific genes, through the methylation of CpGs, and compatible with the concept of the control of gene activation by the balance between this global negative control and activators acting on chromatin structure.

If the repression of the CpG righ LacZ gene reflects the global negative control of the genome, then, in addition to embryonic and somatic cells, other stages also undergo this control including: the extra-embryonic cells, the stem cells of male germ cells (both gonocytes and spermatogonies) and the female germ cells at Pl-Lp-Zy and diplotene stages. Consequently, the negative control of the genome would be always associated with the activity of specialized cells, excluding only multipotential cells of cleavage embryos.

At two periods during the life of the organism, cells seem not to enforce this negative control: at the morula-blastocyst stage, at the pachytene stage during oogenesis and the corresponding stage during spermatogenesis. However, the relief from negative control in these cells does not appear to be mediated by the trans-acting elements of the repressive complex, but through the demethylation of CpGs. It would seem easier and more efficient to relieve the genic repression at these stages by temporally inhibiting the expression of one or more components of the repressor complex than to modify all CpGs of the genome. Therefore, cyclic demethylation of the genome is probably necessary for more than merely the specific gene activation in cells at these stages of development.

Both the maintenance of a global negative regulatory mechanism and the maintenance of a periodic demethylation are apparently crucial for the organism. However, the maintenance of a repressive mechanism based on DNA methylation represents a heavy genetic and epigenetic load for both the genome (through germ cells) and the organism (through somatic cells). The properties of EFLagZ illustrates this point because even though the CpG density of this transgene is close to that of CpG poor endogenous genes, it was still inhibited, particularly in somatic cells, suggesting that this repression can still act on CpG poor endogenous genes. Similarly, tissue-specific genes that contain CpG rich regions, would also be particularly susceptible to this repressive mechanism. Because the methylation pattern is clonally transmitted, repression of these genes would be maintained and accumulated in daughter cells. The general hypomethylation of the genome at the beginning of embryogenesis therefore, may serve to counteract the repression of genes. The consequence of this hypomethylation is an immediate gain for the embryo and the organism and a genetic gain, through germ cells for the next generation. The general methylation which follows the demethylation occurs in tens or hundreds of individual cells (Warnecke and Clark, 1999). This polyclonal event is also advantageous to the organism, since potentially inappropriate inactivation caused by this remethylation will not affect every cell of the embryo, and cells with an incorrect pattern of methylation can be ultimately eliminated.

On the other hand, an extended period of genomic hypomethylation could potentially cause cellular disorders (Foss et al., 1993) (Finnegan et al., 1996; Kakutani et al., 1996) and this could explain why a rapid methylation follows demethylation at the blastocyst stage and why the female genome methylates at the diplotene stage prior to the growth phase. In this latter case, methylation might also be needed to prevent the inappropriate expression of genes whose products could accumulate in the egg and possibly be maternally transmitted to the embryo.

Ubiquitous genes may have evolved towards a lower CpGs content in response to the maintenance of this global, CpG-dependent, negative control system. In support of this idea is the fact that 82% of genes with a broad expression lack CpG islands outside of their promotors (Larsen et al., 1992). This might allow them to escape the activator/repressor system of regulation. Tissue-specific genes probably evolved towards a more refined activating mechanism, involving cis and trans-activators, to overcome this CpG-dependent repression. Indeed, it has been shown that one function of the transcriptional machinery is to modify chromatin into an active conformation (Struhl, 1996). It fits with the concept of gene activity based on a balance between global negative control and activators acting on chromatin structure. It appears paradoxical therefore, that most tissue-specific genes have preserved at least one CpG ricn region, usually located outside of the promotor (Larsen et al., 1992). Our observations suggest that this CpG rich region could be used to inhibit tissue-specific gene activity through a general mechanism, particularly at developmental stages where negative control of such genes is essential, such as the period of tissue diversification at about 8 dpc. In this regard it is interesting to note that the null mutants for methyltransferase (dnmt 1) or MeCP2 exhibit lethality at this stage (Li et al., 1992; Tate, et al., 1996).

To conclude, all these observations suggest that the mammalian genome is not simply controlled by activating it above basal levels but is also actively repressed. Such a system may permit more discrete regulation and a large range of gene activity levels through the combined activity of activators and repressors. Such a fine tuning mechanism with respect to gene activity could, in turn, result in elaboration of more complex regulatory networks. Other functions generally associated with methylation in mammals are the control over the spreading of repeated sequences of transposons and genomic imprinting (Walsh and Bestor, 1999), and there might constitute some secondary uses of this more fundamental mechanism.

### Table 1. HPRTLacZDCR transgene expression in gonads during development

Embryos or animals were obtained by crossing transgenic males or females with (B6D2) F1 animals to analyse paternal (bottom table) and maternal (top table) transgene expression. Gonads were recovered at different stages of development and satined with X-gal. Expression in germ cells is depicted as follows: - : β-gal - cells; + : β-gal+ cells and γ: few β-gal+ cells. Numbers between arrows represent the total number of male or female embryo examined.

1: five transgenic females were β-gal+ in gonads; 2: one transgenic male was β-gal+ in gonads; 3: one male was β-gal+ in gonads; 4: five transgenic males were β-gal+ in gonads and nd: not determined. The last column of the bottom table refers to quantitative expression of the paternal transgene in adult testis. The β-gal activity was quantified with the fluorogenic substrate of β-galactosidase, MUG. β-gal activity of control testis was 41.5 x 10⁻⁷ β-gal units measured with a mean value of 12 control testes.

### Table 2. Expression of paternally and maternally transmitted DCR and HPRTLacZ transgenes during early development

Transgenic males or females were mated with (B6D2) F1 animals to analyse paternal and maternal transgene expression. All the transgenic mice used were homozygous except for DCR4 mice.

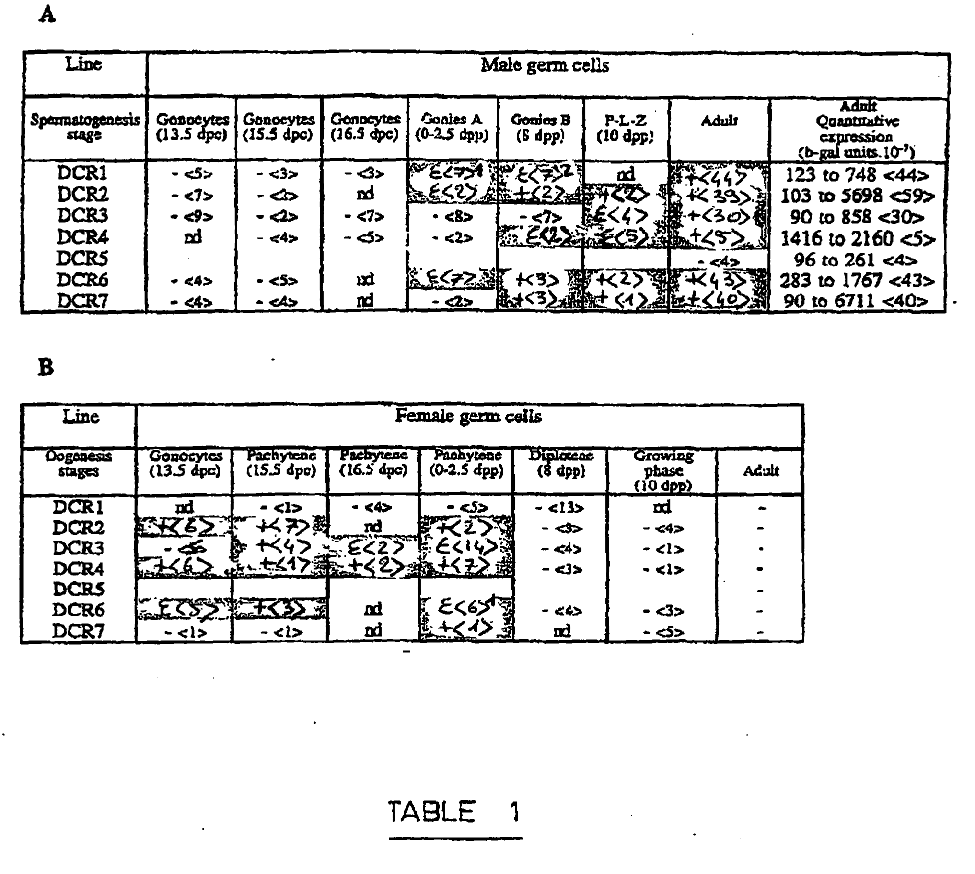

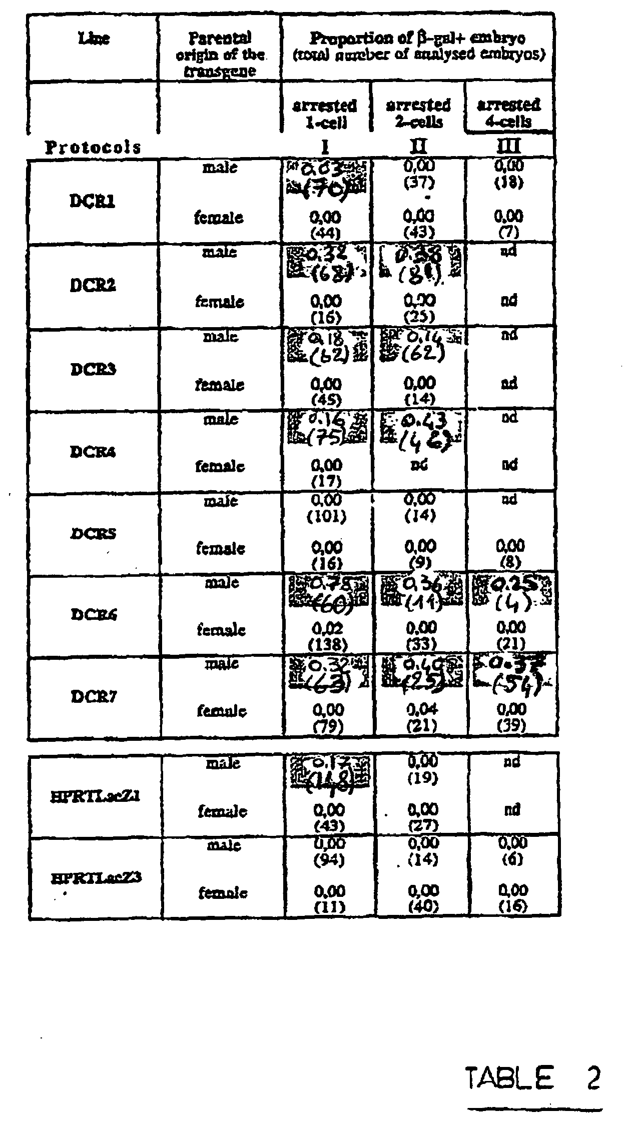

### REFERENCES:

Throughout this paper, reference is made to a number of articles of scientific literature. Each of such papers are hereby incorporated in their entirety by such reference. Those articles which can be found in the technical literature are listed below:
- Ariel, M., McCarrey, J. and Cedar, H. (1991). Methylation patterns of testis-specific genes, Proc Natl Acad Sci USA 88, 2317-2321
- Bestor, T.H. (1998). Gene silencing. Methylation meets acetylation [news; comment]. Nature 393, 311-312
- Bird, A. (1992). The essentials of DNA methylation. Cell 70, 5-8
- Bird, A.P. (1995). Gene number, noise reduction and biological complexity [see comments]. Trends Genet 11, 94-100
- Bonnerot, C., Grimber, G., Briand, P. and Nicolas, J.F. (1990). Patterns of expression of position-dependent integrated transgenes in mouse embryo. Proc Natl Acad Sci US 87, 6331-6335
- Bonnerot, C and Nicolas, J.-F. (1992). Manipulation of expression of a position-dependent transgene by the β-globin locus control region. Submitted to Nature
- Bonnerot, C. and Nicolas, J.-F. (1993). Application of LacZ gene fusions to post-implantation development. In Methods in Enzymology: Guide to techniques in mouse development, vol. 225 (ed. A. Press), pp. 451-469. SanDiego (California): Wassarman, P.M. and DePamphilis, M.L.
- Bonnerot, C. and Nicolas, J.F. (1993a). Lineage control of an integration site-dependent transgene combined with the β-globin locus control region. C.R. Acad. Sci. Paris 316, 352-357
- Bonnerot, C., Rocancourt, D., Briand, P, Grimber, G. and Nicolas, J.F. (1987). A β-galactosidase hybrid protein targeted to nuclei as a marker for developmental studies. Proc. Natl. Acad. Sci. 84, 6795-6799
- Boyes, J. and Bird, A. (1991). DNA methylation inhibits transcription indirectly via a methyl-CpG binding protein. Cell 64, 1123-1134
- Boyes, J. and Bird, A. (1992). Repression of genes by DNA methylation depends on CpG density and promotor strength: evidence for involvement of a methyl-CpG binding protein. EMBO J 11, 327-333
- Brownell, J.E. and Alils, C.D. (1996). Special HATs for special occasions: linking histone acetylation to chromatin assembly and gene activation. Current Opinion in Genetics & Development 6,176-184
- Buschhausen, G., Wittig, B., Graessmann, M. and Graessmann, A. (1987). Chromatin structure is required to block transcription of the methylated herpes simplex virus thymidine kinase gene. Proc Natl Acad Sci USA 84, 1177-1181
- Cameron, E.E., Bachman, K.E., Myohanen, S., Herman, J.G. and Baylin, S.B. (1999). Synergy of demethylation and histone deacetylase inhibition in the re-expression of genes silenced in cancer. Nat Genet 21, 103-107
- Chaillet, J.R. (1994). Genomic imprinting: lessons from mouse transgenes. Mutat Res 307, 441-449
- Collas, P. (1998). Modulation of plasmid DNA methylation and expression in zebrafish embryos. Nucl Acids Res 26, 4454-4461
- Finnengan, E.J., Peacock, W.J. and Dennis, E.S. (1996). Reduced DNA methylation in Arabidopsis thaliana results in abnormal plant development. Proc Natl Acad Sci USA 93, 8449-8454
- Forlani, S., Montfort, L. and Nicolas, J. (1998). Application of transgenes and transgenic mice to study of gene activity from the oocyte to early embryo. In Microinjections and Transgenesis: Strategies and Protocols. (ed. A. Cid-Arregui and A. Garcia-Carranca), pp. 369-412. Heidelberg: Springer-Veriag
- Forlani, S. and Nicolas, J. (1996). Quantification of minute levels of B-galactosidase. The example of individual 2 to 16-cell stage mouse embryos. Trends Genet 12, 498-500
- Foss, H.M., Roberts, C.J., Claeys, K.M. and Selker, E.U. (1993). Abnormal chromosome behavior in Neurospora mutants defective in DNA methylation [published erratum appears in Science 1995 Jan 20; 267(5196):316]. Science 262, 1737-1741
- Gardner, R.L. and Lawrence, P.A. (1985). Single cell marking and cell lineage in animal development. Phil Trans R Soc Lond B312, 1-187
- Ghazi, H., Gonzales, F.A. and Jones, P.A. (1992). Methylation of CpG-island-containing genes in human sperm, fetal and adult tissues. Gene 114, 203-210
- Goto, T., Christians, E. and Monk, M. (1998). Expression of an Xist promotor-luciferase construct during spermatogenesis and in preimplantation embryos: regulation by DNA methylation. Mol Reprod Dev 49, 356-367
- Goto, T. and Monk, M. (1998). Regulation of X-chromosome inactivation in development in mice and humans. Microbiol Mol Biol Rev 62, 362-378
- Groudine, M. and Conkin, K.F. (1985). Chromatin structure and de novo methylation of sperm DNA: implications for activation of the paternal genome. Science 228, 1061-1068
- Hanaoka, K., Hayasaka, M., Uetsuki, T., Fujisawa-Sehara, A. and Nabeshima, Y. (1991). A stable cellular marker for the analysis of mouse chimeras: the bacterial chloramphenicol acetyltransferase gene driven by the human elongation factor 1 alpha promotor. Differentiation 48, 183-189
- Hendrich, B. and Bird, A. (1998). Identification and characterization of a family of mammalian methyl-CpG binding proteins. Mol Cell Biol 18, 6538-6547
- Hogan, B., Costantini, F. and Lacy, E. (1986). Manipulating the mouse embryo: A laboratory manual (ed. New York)
- Howell, C.Y., Steptoe, A.L, Miller, M.W. and Chaillet, J.R. (1998). cis-Acting signal for inheritance of imprinted DNA methylation patterns in the preimplantation mouse embryo. Mol Cell Biol 18, 4149-4156
- Hsieh, C.L. (1994). Dependence of transcriptional repression on CpG methylation density. Mol Cell Biol 14, 5487-5494
- Jones, P.L., Veenstra, C.J., Wade, P.A., Vermaak, D., Kass, S.U., Landsberger, N., Strouboulis, J. and Wolffe. A.P. (1998). Methylated DNA and MeCP2 recruit histone deacetylase to repress transcription. Nat Genet 19, 187-191
- Kafri, T., Ariel, M., Brandeis, M., Shemer, R., Urven, L. and McCarrey, J. (1992). Developmental pattern of gene-specific DNA methylation in the mouse embryo and germ line. Genes Dev 6, 705-714
- Kakutani, T., Jeddeloh, J.A., Flowers, S.K., Munakata, K. and Richards, E.J. (1996). Developmental abnormalities and epimutations associated with DNA hypomethylation mutations. Proc NatI Acad Sci USA 93. 12406-12411
- Kass, S.U., Landsberger, N. and Wolffe, A.P. (1997). DNA methylation directs a time-dependent repression of transcription Initiation. Current Biol 7, 157-165
- Kim, D.W., Uetsuki, T., Kaziro, Y., Yamaguchi, N. and Sugano, S. (1990). Use of the human elongation factor 1 alpha promotor as a versatile and efficient expression system. Gene 91, 217-223
- Klarsfeld, A., Bessereau, J.-L., Salmon, A.-M., Trifler, A., Babinet. C. and Changeux, J.-P. (1991). An acetylcholine receptor alpha-subunit promotor conferring preferential synaptic expression in muscle of transgenic mice. EMBO J 10, 625-632
- Kluin, P.M. and de Rooij, D.G. (1981). A comparison between the morphology and cell kinetics of gonocytes and adult type undifferentiated spermatogonia in the mouse. Int J Andrology 4, 475-493
- Komura, J., Okada, T. and Ono T. (1995). Repression of transient expression by DNA methylation in transcribed regions of reporter genes introduced into cultured human cells. Biochim Biophys Acta 1260, 73-78
- Kress, C., Vogels, R., de Graaff, W., Bonnerot, C., Hameleers, M., Nicolas, J.F. and Deschamps, J. (1990). Hox-2.3 upstream sequences mediate spatiotemporally restricted LacZ expression in intermediate mesoderm derivatives of transgenic mice. Development 109, 775-786
- Kudo, S. (1998). Methyl-CpG-binding protein MeCP2 represses Sp1-activated transcription of the human leukosialin gene when the promotor is methylated Moi Cel Biol 18, 5492-5499
- Larsen, F., Gundersen G., Lopez, R. and Prydz, H. (1992). CpG islands as gene markers in the human genome, Genomics 13, 1095-1107
- Li, E., Bestor, T.H. and Jaenisch, R. (1992). Targeted mutation of the DNA methyltransferase gene results in embryonic lethality. Cell 69, 915-926
- Martin, C.C., Laforest, L., Akimenko, M.A. and Ekker, M. (1999). A role for DNA methylation in gastrulatian and somite patterning. Dev Biol 206, 189-205
- Monk, M., Boubelik, M. and Lehnert, S. (1987). Temporal and regional changes in DNA methylation in the embryonic, extraembryonic and germ cell lineages during mouse embryo development. Development 99, 379-382
- Nan, X., Campoy, F.J. and Bird, A. (1997). MeCP2 is a transcriptional repressor with abundant binding sites in genomic chromatin. Cell 88, 471-481
- Nan, X., Ng, H.H., Johnson, C.A., Laherty, C.D., Turner, B.M., Eisenman, R.N. and Bird. A. (1998). Transcriptional repression by the methyl-CpG-binding protein MeCP2 involves a histone deacetylase complex [see comments]. Nature 393, 386-389
- Nan, X., Tate, P., Li, E. and Bird, A (1996). DNA methylation specifies chromosomal localization of MeCP2. Mol Cell Biol 16, 414-421
- Rougier, N., Boure'his, D., Gomes, D.M., Niveleau, A., Plachot, M. Paldi, A. and Viegas-Pectuignot, E. (1998). Chromosome methylation patterns duringt mammalian preimplantation development. Genes Dev 12, 2108-2113
- Salvatore, P., Benvenuto, G., Caporaso, M., Bruni, C.B. and Chiariotti, L. (1998). High resolution methylation analysis of the galectin-1 gene promotor region in expressing and nonexpressing tissues. FEBS Letters 421, 152-158
- Sanford, J.P., Clark, H.J., Chapman, V.M. and Rossant, J. (1987). Differences in DNA methylation during oogenesis and spermatogenesis and their persistence during early embryogenesis in the mouse. Genes Dev 1, 1039-1046
- Schultz, R.M. (1986). In Experimental approaches to mammalian embryonic development. J. Rossant and R.A. Pedersen Ed. Cambridge University Press, 195-227
- Singal, R., Ferris, R., Little, J.A., Wang, S.Z. and Ginder, G.D. (1997). Methylation of the minimal promotor of an embryonic globin gene silences transcription in primary erythroid cells. Proceedings of the National Academy of Sciences of the United States of America 94, 13724-13729
- Struhl, K. (1996). Chromatin structure and RNA polymerase II connection: implications for transcription. Ceii 84, 179-182
- Struhl, K. (1998). Histone acetylation and transcriptional regulatory mechanisms. Genes Dev 12, 599-606
- Talbot, D., Collis, P., Antonicu, M., Vidal, M., Grosveld, F. and Greaves, D.R. (1989). A dominant control region from the human beta-globin locus conferring integration site-independent gene expression. Nature 338, 352-355
- Tate, P., Skarnes, W. and Bird, A. (1996). The methyl-CpG binding protein MeCP2 is essential for embryonic development in the mouse. Nat Genet 12, 205-208
- Trasler, J. M., Hake, L. E., Johnson, P. A., Alcivar, A. A., Millette, C. F. and Hecht, N. B. (1990). DNA methylation and demethylation events during meiotic prophase in the mouse testis. Mol Cell Biol 10, 1828-1834
- Uetsuki, T., Naito, A., Nagata, S. and Kaziro, Y. (1989). Isolation and characterization of the human chromosomal gene for polypeptide chain elongation factor-1 alpha. J Biol Chem 264, 5791-5798
- Vergouwen, R. P., Jacobs, S. G., Huiskamp, R., Davids, J. A. and de Rooij, D. G. (1991). Proliferative activity of gonocytes, Sertoli cells and interstitial cells during testicular development in mice. J Reprod Fert 93, 233-243
- Vemet, M., Bonnerot, C., Briand, P. and Nicolas, J.-F. (1993). Application of LacZ gene fusions to preimplantation development. In Methods in Enzymology: Guide to techniques in mouse development., vol. 225 (ed. 434-451: Wassarwan, P. M., DePamphilis, M. L.
- Walsh, C. P. and Bestor, T. H. (1999). Cytosine methylation and mammalian development. Genes Dev 13, 26-34
- Warnecke, P. M. and Clark, S. J. (1999). DNA methylation profile of the mouse skeletal alpha-actin promotor during development and differentiation. Mol Cell Biol 19, 164-172
- Weng. A., Engler, P. and Storb, U. (1995). The bulk chromatin structure of a murine transgene does not vary with its transcriptional or DNA methylation status. Mol Cell Biol 15, 572-579
- Yoshida, M., Horinouchi, S. and Beppu, T. (1995). Trichostatin A and trapoxin: novel chemical probes for the role of histone acetylation in chromatin structure and function. Bioessays 17,423-430
- Zhang, L. P., Stroud, J. C., Walter, C. A., Adrian, G. S. and McCarrey, J. R. (1998). A gene-specific promotor in transgenic mice directs testis-specific demethylation prior to transcriptional activation in vivo. Biol Reprod 59, 284-292

While several embodiments of the invention have been described, it will be understood that the present invention is capable of further modification, and this application is intended to cover any variations. uses, or adaptation of the invention, following in general the principles of the invention and including such departures from the present disclosure as to come within knowledge or customary practice in the art to which the invention pertains, and as may be applied to the essential features hereinbefore set forth and falling within the scope of the invention or the limits of the appended claims.

## Claims

1. An isolated and modified polynucleotide derived from a native eukaryotic gene, the isolated and modified polynucleotide being **characterized in that** it comprises, at the nucleotide level, a modified content of at least one epigenetic motif specific to a prokaryotic host, as compared to the native eukaryotic gene, and being further
**characterized in that:**
- said at least one epigenetic motif consists of 5'GATC3' or of 5'CpG3',
- the content in said motif of the sequence of said isolated and modified polynucleotide has been increased as compared to the content in the same motif of the sequence of said native eukaryotic gene.

2. The isolated and modified polynucleotide of claim 1, **characterized in that** the amino acid sequence of the protein or polypeptide coded by said modified prokaryotic gene is not changed as compared to that coded by said native eukaryotic gene.

3. The isolated and modified polynucleotide of claim 1, **characterized in that** it codes a eukaryotic protein selected from the group consisting of angiogenic proteins, growth factors, cytokines, interleukins and immunoglobulines.

4. An expression vector, **characterized in that** it comprises at least one isolated and modified polynucleotide according to any one of claims 1-3.

5. An isolated host cell, **characterized in that** it is transformed with the expression vector of claim 4, or **in that** it has been genetically modified as to comprise and/or express an isolated and modified polynucleotide according to any one of claims 1-3.

6. The cell of claim 5, **characterized in that** it has been genetically modified using a method selected from the group consisting of bacterial transformation, transgenesis, stem cell transformation, viral transfection and artificial chromosome insertion.

7. The isolated polynucleotide of claim 1, for use to express the protein or polypeptide it encodes in a prokaryotic host cell.

8. A method to increase the capacity of an isolated native eukaryotic gene of being expressed in a prokaryotic host, **characterized in that** it comprises modifying the nucleotide sequence of said isolated native eukaryotic gene by increasing its content in 5'GATC3' or of 5'CpG3', without changing the amino acid sequence of the protein or polypeptide coded by said native eukaryotic gene.
